# EUROPEAN PATENT APPLICATION

(11) **EP 4 035 747 A1**
(43) Date of publication of application: **03.08.2022**
(21) Application number: 22154736.7
(22) Date of filing: 02.02.2022
(51) Int. Cl.: A63B 22/06, A63B 71/06

(54) **EXERCISE SYSTEM**

(30) Priority: 02.02.2021 JP 2021015234; 24.09.2021 JP 2021155764
(71) Applicant: Yamaha Hatsudoki Kabushiki Kaisha, Iwata-shi, Shizuoka-ken 438-8501 (JP)
(72) Inventor: Shiraishi, Shoji, Shizuoka (JP); Fujii, Hisashi, Shizuoka (JP); Oishi, Hisashi, Shizuoka (JP)
(74) Representative: Grünecker Patent- und Rechtsanwälte PartG mbB

(57) **Abstract**

In an exercise system 1, a first user 10 who uses an exercise device 11 in an indoor place 70 and a second user 20 who moves outdoors do exercise simultaneously. The exercise system 1 includes: for the first user 10, an exercise device 11 that is used by the first user 10 in the indoor place 70; and a first processing device 101 that calculates a virtual movement distance of the first user 10; and for the second user 20, a second processing device 201 that obtains positional information indicating geographic coordinates of a position of the second user 20 who moves outdoors. The first processing device 101 displays, on a display device 105, an image 80, 90, 250 including a virtual positional relationship between the first user 10 and the second user 20 in an outdoor area where the second user 20 is moving, based on the positional information of the second user 20 and the virtual movement distance of the first user 10.

## Description

The present invention relates to exercise systems using an exercise device.

Among the exercise devices for maintaining and promoting health are, for example, a virtual cycling device capable of allowing the user to enjoy virtual cycling indoors (see, for example, JP 2005-021391 A).

In the virtual cycling device disclosed in JP 2005-021391 A, a bicycle is mounted on a dedicated roller stage, and a display device is disposed in front of the bicycle. The display device displays previously prepared images of landscapes and road surfaces, etc.

By rotating the pedals of the bicycle while viewing images displayed on the display device, the user can have a virtual experience as if the user were cycling outdoors.

It is the object the present invention to provide an exercise system providing a more realistic experience to the user when the user is doing exercise. According to the present invention said object is solved by an exercise system having the features of independent claim 1. Preferred embodiments are laid down in the dependent claims.

An embodiment of the present teaching provides an exercise system in which a first user who uses an exercise device indoors and a second user who moves outdoors do exercise simultaneously, including: for the first user, an exercise device that is used by the first user indoors; a first sensor that outputs a signal corresponding to an operation of the exercise device produced by the first user using the exercise device; a first processing device configured to calculate a virtual movement distance of the first user based on the output signal of the first sensor; and a first display device configured to present information to the first user; and for the second user, a second processing device configured to obtain and output positional information indicating geographic coordinates of a position of the second user who moves outdoors. The first processing device is configured to display, on the first display device, an image including a virtual positional relationship between the first user and the second user in an outdoor area where the second user is moving, based on the positional information of the second user and the virtual movement distance of the first user.

The first user who does exercise using the exercise device indoors can view the image including the virtual positional relationship between the first user and the second user in an outdoor area where the second user is moving, and therefore, can have a virtual experience as if the first user were moving outdoors together with the second user.

In an embodiment, the first processing device may display, on the first display device, a map showing the position of the second user and a virtual position of the first user, based on map information of the outdoor area where the second user is moving, the positional information of the second user, and the virtual movement distance of the first user.

The first user can recognize the virtual positional relationship between the first user and the second user in the outdoor area by viewing the map indicating the position of the second user and the virtual position of the first user.

In an embodiment, the first processing device may obtain image information indicating landscape-related images related to landscapes at a plurality of positions in the outdoor area, calculate a virtual position of the first user in the outdoor area from the virtual movement distance of the first user, and display, on the first display device, a landscape-related image in association with the virtual position of the first user, together with an image of an avatar of the second user whose display position and display size are changed, depending on the virtual positional relationship between the first user and the second user.

Thus, images corresponding to landscapes that could be viewed by the first user if the first user were actually moving outdoors are displayed on the first display device, whereby a more realistic experience can be provided.

Thus, the position and size of the displayed avatar of the second user are changed, whereby the first user can intuitively recognize the virtual positional relationship between the first user and the second user.

In an embodiment, the second processing device may output image information of landscapes of the outdoor area captured during movement of the second user. The first processing device may display an image of a landscape associated with the virtual position of the first user on the first display device.

Thus, an image of a current landscape associated with the virtual position of the first user is displayed on the first display device, whereby a more realistic experience can be provided.

In an embodiment, the first processing device may display, on the first display device, an image of an avatar of the second user whose display position and display size are changed, depending on the virtual positional relationship between the first user and the second user.

Thus, the position and size of the displayed avatar of the second user are changed, whereby the first user can intuitively recognize the virtual positional relationship between the first user and the second user.

In an embodiment, the second user may move on a preset travel route. The first processing device may calculate a virtual position of the first user on the preset travel route from the virtual movement distance of the first user.

As a result, the virtual position of the first user can be easily calculated.

In an embodiment, the exercise device used by the first user may be for indoor cycling.

Thus, the first user who is present indoors can have a virtual experience as if the first user were moving outdoors together with the second user while riding a bicycle.

In an embodiment, the second user may move outdoors while riding a bicycle.

Thus, the first user who is present indoors can have a virtual experience as if the first user were cycling outdoors together with the second user.

In an embodiment, the exercise device may be provided with an adjustment device configured to adjust the magnitude of a load applied to the first user. The first processing device may obtain slope angles of road surfaces at a plurality of positions on a travel route on which the second user moves, calculate a virtual position of the first user on the travel route from the virtual movement distance of the first user, and control the adjustment device such that the load applied to the first user is changed, depending on the slope angle at a predetermined position on the travel route associated with the virtual position of the first user.

Thus, the first user virtually experiences a load that would be applied if the first user were actually traveling outdoors, whereby a more realistic experience can be provided.

In an embodiment, the bicycle that the second user rides may be provided with an angular sensor that outputs a signal corresponding to an inclination of the bicycle. The second processing device may calculate a slope angle of the travel route on which the second user is moving, from the output signal of the angular sensor, and output slope angle information indicating the calculated slope angle. The first processing device may obtain a slope angle at the predetermined position from the slope angle information.

Thus, the first user can virtually experience a load that is applied to the second user when the second user is currently traveling outdoors, whereby a more realistic experience can be provided.

In an embodiment, the positional information of the second user may include altitude information indicating an altitude of the position of the second user. The first processing device may calculate a length between two predetermined positions on the travel route on which the second user moves, based on geographic coordinates of the two predetermined positions, calculate an altitude difference between the two predetermined positions based on the altitude information of the two predetermined positions, and calculate a slope angle of the road surface based on the calculated length and altitude difference.

Thus, the slope angle of a road surface is calculated using the altitude information, whereby the value of the slope angle can be obtained without using the angular sensor.

In an embodiment, the exercise device may be provided with an adjustment device configured to adjust the magnitude of a load applied to the first user. The first processing device may control the adjustment device such that the load applied to the first user is smaller when the first user is positioned behind the second user than when ahead of the second user in the virtual positional relationship between the first user and the second user.

Thus, the load applied to the first user is reduced to a greater extent when the first user is positioned behind the second user, and therefore, the first user can virtually experience slipstreaming, whereby a more realistic experience can be provided.

In an embodiment, the exercise device may be provided with a saddle on which the first user sits, a handlebar that is held by the hands of the first user, and a vibration generation device that vibrates at least one of the saddle and the handlebar. The first processing device may obtain vibration information associated with a plurality of positions on a travel route on which the second user moves, calculate a virtual position of the first user on the travel route from the virtual movement distance of the first user, and control the vibration generated by the vibration generation device, based on the vibration information of a predetermined position on the travel route associated with the virtual position of the first user.

Thus, the first user virtually experiences vibration that would occur if the first user were actually traveling outdoors, whereby a more realistic experience can be provided.

In an embodiment, the bicycle that the second user rides may be provided with a vibration sensor that outputs a signal corresponding to vibration of the bicycle. The second processing device may generate vibration sensor output information from the output signal of the vibration sensor, and outputs the vibration sensor output information. The first processing device may associate the vibration sensor output information with the positional information of the second user to generate the vibration information.

Thus, the first user can virtually experience vibration that is felt by the second user when the second user is currently traveling outdoors, whereby a more realistic experience can be provided.

In an embodiment, a blower that creates airflow may be provided for the first user. The bicycle of the second user may be provided with a wind speed sensor that outputs a signal corresponding to wind striking the bicycle of the second user or the second user. The second processing device may generate wind speed information from the output signal of the wind speed sensor, and outputs the wind speed information. The first processing device may control the airflow created by the blower, based on the wind speed information.

Thus, the first user can virtually experience wind that is felt by the second user when the second user is currently traveling outdoors, whereby a more realistic experience can be provided.

In an embodiment, the exercise device may be provided with an adjustment device configured to adjust the magnitude of a load applied to the first user. The bicycle of the second user may be provided with a wind speed sensor that outputs a signal corresponding to wind striking the bicycle of the second user or the second user. The second processing device may generate wind speed information from the output signal of the wind speed sensor, and output the wind speed information. The first processing device may control the adjustment device such that the magnitude of the load applied to the first user is changed, depending on the wind speed information.

Thus, the first user can virtually experience a load corresponding to wind that is felt by the second user when the second user is currently traveling outdoors, whereby a more realistic experience can be provided.

In an embodiment, the second processing device may display information that is presented to the second user on a second display device. The first processing device may output information about the virtual movement distance of the first user. The second processing device may calculate a virtual position of the first user in the outdoor area based on the information about the virtual movement distance of the first user, and display an image showing the virtual position of the first user on the second display device.

Thus, the second user can compare their own position with the virtual position of the first user in the outdoor area by viewing the image including the virtual position of the first user. As a result, the second user can experience as if the second user moved outdoors together with the first user.

In an embodiment, the second processing device may display information that is presented to the second user on a second display device. The first processing device may calculate a virtual position of the first user in the outdoor area based on the virtual movement distance of the first user, and output information about the virtual position of the first user. The second processing device may display an image showing the virtual position of the first user on the second display device based on the information about the virtual position of the first user.

Thus, the second user can compare their own position with the virtual position of the first user in the outdoor area by viewing the image including the virtual position of the first user. As a result, the second user can experience as if the second user moved outdoors together with the first user.

In an embodiment, the first processing device and the second processing device may each include a voice communication device. The first user and the second user may be allowed to have a voice communication through the first processing device and the second processing device.

Thus, the first user and the second user can do exercise while having a conversation with each other.

In an embodiment, the exercise device used by the first user may be provided with an adjustment device configured to adjust the magnitude of a load applied to the first user. The first processing device may control the adjustment device such that the magnitude of the load applied to the first user is changed, depending on a state in a game that is played by the first user and the second user.

Thus, the load applied to the first user is changed, depending on a state in a game, whereby the first user can do exercise while enjoying playing the game.

In an embodiment, the bicycle that the second user rides may be provided with an adjustment device configured to adjust the magnitude of a load applied to the second user. The second processing device may control the adjustment device provided on the bicycle such that the magnitude of the load applied to the second user is changed, depending on a state in a game that is played by the first user and the second user.

Thus, the load applied to the second user is changed, depending on a state in a game, whereby the second user can do exercise while enjoying playing the game.

In an embodiment, the preset travel route may include a curved portion. For the curved portion, an upper limit value of travel speed may be previously set. The first processing device may perform control such that a virtual travel speed of the first user is lower than or equal to the upper limit value when a virtual position of the first user is on the curved portion.

The speed at which traveling is possible on a curved portion of a road has an upper limit. The speed at which traveling is possible on a curved portion is, for example, previously set based on the radius of curvature of the curved portion.

The virtual travel speed of the first user on a curved portion is set to be lower than or equal to a preset upper limit value. For example, when the virtual position of the first user is moved from a straight line portion to a curved portion, then if the virtual travel speed is greater than the upper limit value, the virtual travel speed is controlled to be lower than or equal to the upper limit value. As a result, the first user can have an experience similar to traveling on an actual curve, whereby a more realistic experience can be provided.

The virtual position of the first user can be inhibited from being moved on a curved portion at a travel speed that would cause the first user to go off that curved portion if the first user were traveling on an actual road. Thus, the first user can have an experience similar to traveling on an actual curve, whereby a more realistic experience can be provided.

When the first user who is present indoors and the second user who is present outdoors compete against each other in a race, then if the virtual travel speed of the first user is reduced on curved portions where deceleration is required, the fairness of the race can be increased.

In an embodiment, the preset travel route may include a first curved portion and a second curved portion. A radius of curvature of the second curved portion may be smaller than a radius of curvature of the first curved portion. A preset upper limit value of travel speed of the second curved portion may be smaller than a preset upper limit value of travel speed of the first curved portion.

Thus, the upper limit value of travel speed is reduced to a greater extent for a curved portion having a smaller radius of curvature, and therefore, the first user can have an experience similar to traveling on actual curves, whereby a more realistic experience can be provided.

In an embodiment, the preset travel route may include a straight line portion and a curved portion. The first processing device may set a virtual travel speed of the first user with respect to an operation amount of the exercise device to be lower when a virtual position of the first user is on the curved portion than when on the straight line portion.

In the case of traveling on actual roads, the travel speed is typically lower on curves than on straight lines. If the virtual travel speed with respect to the operation amount of the exercise device is smaller when the first user is virtually positioned on curved portions than on straight line portions, the first user can have an experience similar to a change in speed during traveling on an actual road.

In an embodiment, the exercise device may be provided with an adjustment device configured to adjust the magnitude of a load applied to the first user. The preset travel route may include a straight line portion and a curved portion. The first processing device may control the adjustment device such that the load applied to the first user is greater when the position of the second user is on the curved portion than when on the straight line portion.

The second user who travels on an actual outdoor route may take a lower travel speed on curves than on straight lines. When the first user who is present indoors and the second user who is present outdoors compete against each other in a race, then if the load applied to the first user is increased so that the second user does not suffer from a disadvantage when the second user decelerates on curved portions, the fairness of the race can be increased.

In an embodiment, the exercise device may be provided with an adjustment device configured to adjust the magnitude of a load applied to the first user. For the preset travel route, the total amount of loads applied to the first user may be previously set. The first processing device may control the adjustment device such that the total amount of loads that are applied to the first user when a virtual position of the first user moves from the start to finish of the preset travel route is equal to a predetermined total load amount.

The timing at which a load is applied to the first user for increase of fairness may not be particularly limited. Therefore, the application of a load to the first user can be inhibited from being performed at a timing unnatural to the first user.

An embodiment of the present teaching provides a method for providing exercise that a first user who uses an exercise device indoors and a second user who moves outdoors do simultaneously, including: calculating a virtual movement distance of the first user based on an operation of the exercise device produced by the first user using the exercise device indoors; obtaining positional information indicating geographic coordinates of a position of the second user who moves outdoors; and displaying an image including a virtual positional relationship between the first user and the second user in an outdoor area where the second user is moving, based on the positional information of the second user and the virtual movement distance of the first user.

The first user who does exercise using the exercise device indoors can view the image including the virtual positional relationship between the first user and the second user in an outdoor area where the second user is moving, and therefore, can have a virtual experience as if the first user were moving outdoors together with the second user.

An embodiment of the present teaching provides a computer program for causing a computer to provide exercise that a first user who uses an exercise device indoors and a second user who moves outdoors do simultaneously. The computer program causes the computer to execute: calculating a virtual movement distance of the first user based on an operation of the exercise device produced by the first user using the exercise device indoors; obtaining positional information indicating geographic coordinates of a position of the second user who moves outdoors; and displaying an image including a virtual positional relationship between the first user and the second user in an outdoor area where the second user is moving, based on the positional information of the second user and the virtual movement distance of the first user.

The first user who does exercise using the exercise device indoors can view the image including the virtual positional relationship between the first user and the second user in an outdoor area where the second user is moving, and therefore, can have a virtual experience as if the first user were moving outdoors together with the second user.

According to an embodiment of the present teaching, provided is an exercise system in which a first user who uses an exercise device indoors and a second user who moves outdoors do exercise simultaneously. The first user who does exercise using the exercise device indoors can view an image including a virtual positional relationship between the first user and the second user in an outdoor area where the second user is moving, and therefore, can have a virtual experience as if the first user were moving outdoors together with the second user.

### BRIEF DESCRIPTION OF THE DRAWINGS

FIG. 1 is a schematic diagram illustrating an example of a configuration of an exercise system 1 according to an embodiment of the present teaching.
FIG. 2 is a block diagram illustrating examples of hardware configurations of a first user's terminal device 100 and a bicycle trainer 60 according to an embodiment of the present teaching.
FIG. 3 is a block diagram illustrating an example of a hardware configuration of a second user's terminal device 200 according to an embodiment of the present teaching.
FIG. 4 is a block diagram illustrating an example of a hardware configuration of a server 30 according to an embodiment of the present teaching.
FIG. 5 is a flowchart illustrating an example of a process of generating an image including a virtual positional relationship between a first user 10 and a second user 20 according to an embodiment of the present teaching.
FIG. 6 is a diagram illustrating an example of an image including a virtual positional relationship between a first user 10 and a second user 20 according to an embodiment of the present teaching.
FIG. 7 is a diagram illustrating an example of an enlarged image of a portion of an altitude graph 90 according to an embodiment of the present teaching.
FIG. 8 is a diagram illustrating another example of an image including a virtual positional relationship between a first user 10 and a second user 20 according to an embodiment of the present teaching.
FIG. 9 is a diagram illustrating an avatar 251 when a second user 20 is located at a position that is a short distance in front of ta first user 10 according to an embodiment of the present teaching.
FIG. 10 is a diagram illustrating an avatar 251 when a second user 20 is located at a position that is a long distance in front of from a first user 10 according to an embodiment of the present teaching.
FIG. 11 is a diagram illustrating an exercise system 1 that provides vibration corresponding to a state of a road surface 86 to a first user 10 according to an embodiment of the present teaching.
FIG. 12 is a diagram illustrating an exercise system 1 that allows a first user 10 to virtually experience wind that is felt by a second user 20 according to an embodiment of the present teaching.
FIG. 13 is a diagram illustrating an example of an image that is displayed on a display device 205 of a second user's terminal device 200 according to an embodiment of the present teaching.
FIG. 14 is a diagram illustrating an exercise system 1 in which a load applied to a second user 20 is changed according to an embodiment of the present teaching.
FIG. 15 is a diagram illustrating an example of a preset travel route 85 according to an embodiment of the present teaching.
FIG. 16 is a diagram illustrating an example of a relationship between the radius of curvature and the upper limit value of travel speed according to an embodiment of the present teaching.

### DETAILED DESCRIPTION

Embodiments of the present teaching will be described in detail below. Some parts of the embodiments may not be described in detail in order to avoid unnecessarily long descriptions. To avoid unnecessarily obscuring the present disclosure, well-known features may not be described or substantially the same features as well-known features may not be redundantly described, for example. This is also for ease of understanding the present teaching.

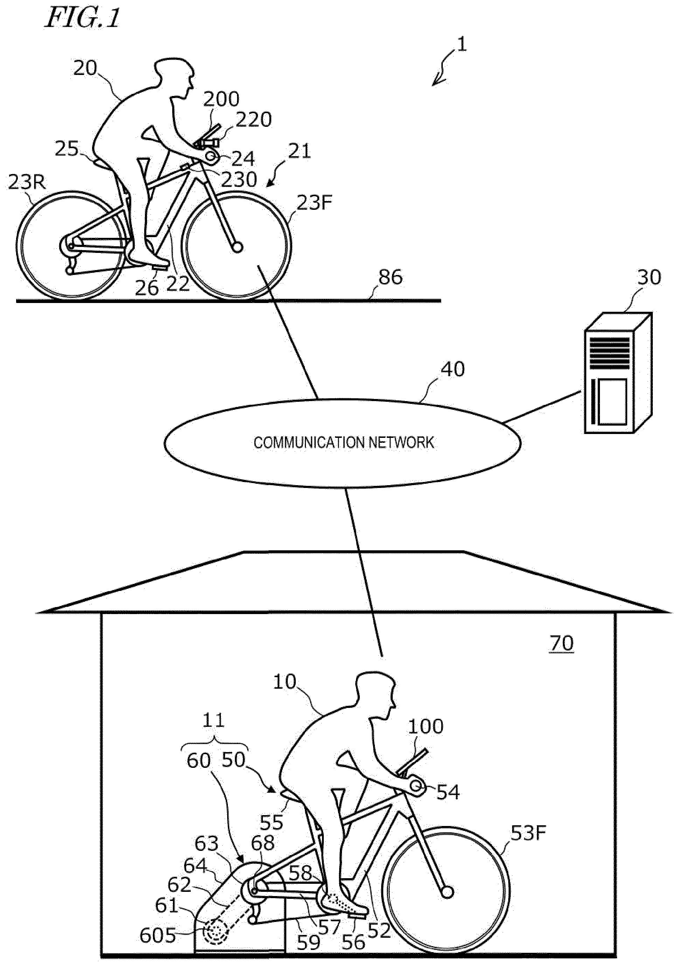

FIG. 1 is a schematic diagram illustrating an example of a configuration of an exercise system 1 according to this embodiment. The exercise system 1 allows a first user 10 who uses an exercise device 11 indoors and a second user 20 who moves outdoors to do exercise simultaneously. As used herein, the term "exercise" should be understood in a broader sense, and is not limited to so-called physical activities performed by the first user 10 and the second user 20. Examples of the exercise performed by the first user 10 and the second user 20 include races (e.g., competitions), touring, tourism, fitness training, and games with physical activities.

The exercise device 11 is provided in an indoor place 70 where the first user 10 is present. The exercise device 11 is, for example, an indoor cycling machine for doing indoor cycling. The second user 20 rides a bicycle 21 on a road surface 86 outdoors, for example.

In the example of FIG. 1, in the exercise device 11, a bicycle trainer 60 is provided for a bicycle 50. The bicycle 50 includes a bicycle frame 52, a front wheel 53F, a handlebar 54, a saddle 55, pedals 56, and a pedal crank shaft 58. The bicycle 50 can be ridden outdoors with a rear wheel attached thereto. Instead of a rear wheel, the bicycle trainer 60 is provided on chain stays 57 of the bicycle 50 of FIG. 1.

The bicycle trainer 60 detects the amount of rotation of the pedals 56 that increases when the first user 10 rotates the pedals 56. The bicycle trainer 60 also adjusts a force that is required for rotation of the pedals 56. In other words, the bicycle trainer 60 adjusts a load that is transmitted from the pedals 56 to the first user 10 when the first user 10 rotates the pedals 56. The bicycle trainer 60 also supports the bicycle 50 so that the bicycle 50 is maintained in an upright position.

The type of the bicycle trainer 60 is not particularly limited. For example, examples of the type of the bicycle trainer 60 include direct drive, tire drive, three rollers, and bike-integrated trainer. The bicycle trainer 60 of FIG. 1 is a direct-drive trainer.

The bicycle trainer 60 includes an electric motor 61, a power transmission mechanism 62, a sprocket 63, and a housing 64. The electric motor 61 generates a load that is applied to the first user 10 (hereinafter also referred to as a "torque"). The torque generated by the electric motor 61 is transmitted to the sprocket 63 through the power transmission mechanism 62. The housing 64 houses the electric motor 61 and the power transmission mechanism 62.

The sprocket 63 is rotatably attached to the chain stays 57 through an axle 68. The sprocket 63 is used as a following sprocket of the bicycle 50. A chain 59 is looped around the sprocket 63.

The bicycle 21 that is used by the second user 20 outdoors includes a bicycle frame 22, a front wheel 23F, a rear wheel 23R, a handlebar 24, a saddle 25, and pedals 26. The handlebar 24 is provided with a camera 220. The bicycle frame 22 is provided with an angular sensor 230. The second user 20 can travel on the road surface 86 outdoors, riding the bicycle 21 and rotating the pedals 26.

The handlebar 54 of the bicycle 50 in the indoor place 70 is provided with the first user's terminal device 100. The handlebar 24 of the bicycle 21 located outdoors is provided with the second user's terminal device 200.

The first user's terminal device 100, the second user's terminal device 200, and a server computer 30 (hereinafter denoted by a "server 30") are connected through a communication network 40 so as to communicate with each other. The communication network 40 is, for example, but not limited to, the Internet. The first user's terminal device 100, the second user's terminal device 200, and the server 30 may communicate with each other by wireless communication through mobile telephony or satellite communications.

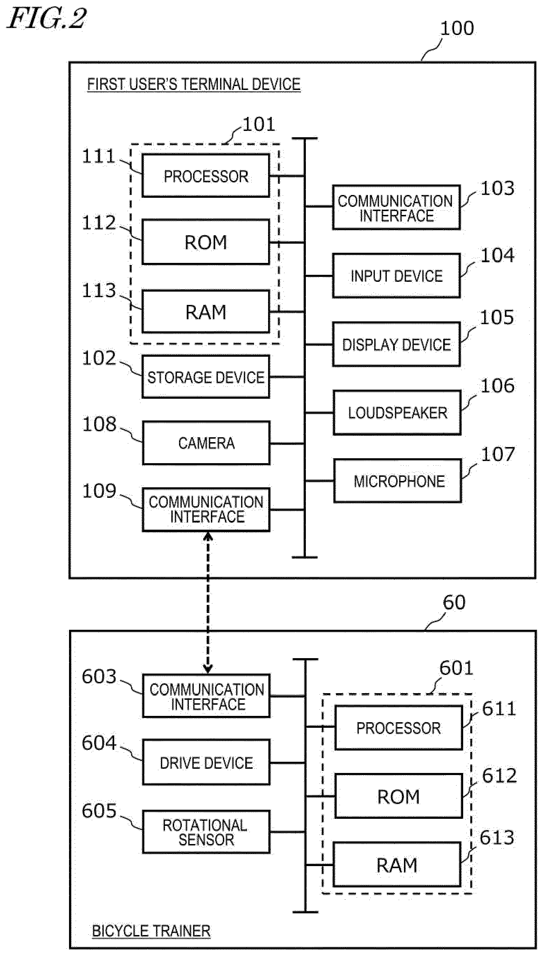

Next, examples of hardware configurations of the first user's terminal device 100 and the bicycle trainer 60 will be described. FIG. 2 is a block diagram illustrating examples of hardware configurations of the first user's terminal device 100 and the bicycle trainer 60.

The first user's terminal device 100 is, for example, a smartphone, tablet computer, or bicycle computer.

The first user's terminal device 100 includes a processing device 101, a storage device 102, a communication interface (first communication device) 103, an input device 104, a display device 105, a loudspeaker 106, a microphone 107, a camera 108, and a communication interface 109. These constituent elements are connected through a bus so as to communicate with each other.

The processing device 101 includes a processor 111, and storage media such as a read only memory (ROM) 112 and a random access memory (RAM) 113. The ROM 112 may store a computer program (or firmware) that causes the processor 111 to execute processes. The computer program may be provided to the server 100 through a storage medium (e.g., a semiconductor memory or optical disc) or electrical communications (e.g., the Internet). Such a computer program may be made commercially available as commercial software.

The processor 111 is a semiconductor integrated circuit such as a central processing unit (CPU). The processor 111 may be implemented by a microprocessor or microcontroller. The processor 111 sequentially executes the computer program stored in the ROM 112, in which instructions for executing various processes are written, to carry out the desired processes.

The processor 111 may be a field programmable gate array (FPGA), graphics processing unit (GPU), application specific integrated circuit (ASIC), or application specific standard product (ASSP) with a CPU mounted thereon, or a combination of two or more selected from these circuits.

The ROM 112 is, for example, a writable memory (e.g., a PROM), a rewritable memory (e.g., a flash memory), or a read-only memory. As described above, the ROM 112 may store a computer program for causing the processor 111 to execute processes. The ROM 112 also stores a computer program that controls operations of the processor 111. The ROM 112 may not necessarily be a single storage medium, or may be a set of storage media. A portion of the set of storage media may be removable.

The RAM 113 provides a work area into which the computer program of the ROM 112 will be temporarily loaded during boot-up. The RAM 113 may not necessarily be a single storage medium, and may be a set of storage media.

The storage device 102 is, for example, a semiconductor storage device, a magnetic storage device, an optical storage device, or a combination thereof. Examples of the semiconductor storage device include solid-state drives (SSDs). Examples of the magnetic storage device include hard disk drives (HDDs). Examples of the optical storage device include optical disk drives and magneto-optical disk (MD) drives.

The communication interface 103 is a communication module for communication with the second user's terminal device 200 and the server 30 through the network 40. The communication interface 103 allows wired communication and/or wireless communication. The communication interface 103 allows wired communication compliant with a communication standard such as USB, IEEE 1394 (registered trademark), or Ethernet (registered trademark). The communication interface 103 allows wireless communication compliant with, for example, the Bluetooth (registered trademark) standard and/or the Wi-Fi (registered trademark) standard. These standards include a wireless communication standard that uses the 2.4 GHz or 5.0 GHz frequency band. The communication interface 103 may be a communication module that allows wireless communication compliant with the Bluetooth Low Energy (BLE) or Low Power Wide Area (LPWA) communication technology. The communication interface 103 may allow wireless communication that uses mobile telephony or satellite communications.

The input device 104 converts instructions from the first user 10 into data, and inputting the data to the computer. The input device 104 is, for example, a touch panel, a keyboard, a mouse, or a combination thereof. The display device 105 is, for example, a liquid crystal display or organic light-emitting diode (OLED) display.

The loudspeaker 106 converts an audio signal into sound, and outputs the sound. The microphone 107 converts sound around the first user's terminal device 100 into an audio signal. The loudspeaker 106 and the microphone 107 can be used as a voice communication device that allows a voice communication with the second user 20. The camera 108 captures an image of a part of surroundings around the first user's terminal device 100 to generate an image signal. For example, the camera 108 can be used to capture an image of the first user 10. The communication interface 109 is a communication module for communication with the bicycle trainer 60.

The bicycle trainer 60 includes a processing device 601, a communication interface 603, a drive device 604, and a rotational sensor 605. These constituent elements are connected through a bus so as to communicate with each other.

The processing device 601 includes a processor 611, a ROM 612, and a RAM 613. The processor 611, the ROM 612, and the RAM 613 are similar to the processor 111, the ROM 112, and the RAM 113 of the first user's terminal device 100 and therefore will not herein be described.

The drive device 604 generates a drive current for driving the electric motor 61 (FIG. 1) according to an instruction from the processor 611. When the electric motor 61 is supplied with the drive current, the electric motor 61 generates a torque. The rotational sensor 605 is provided at any position on a power transmission path through which power generated by drive of the electric motor 61 is transmitted. From another viewpoint, the rotational sensor 605 is provided at any position on a power transmission path between the pedals 56 and the electric motor 61. For example, the rotational sensor 605 may be provided on the axis of rotation of the pedals 56, or on a power transmission path between the electric motor 61 and the sprocket 63. For example, the rotational sensor 605 is provided on the pedal crank shaft 58 which rotatably supports the pedals 56 through crank arms. When the rotational sensor 605 is provided on a power transmission path, the rotational sensor 605 detects the rotation of the power transmission path.

The communication interface 603 is a communication module for communication with the first user's terminal device 100. The communication interfaces 109 and 603 allow wired communication and/or wireless communication. Like the communication interface 103, the communication interfaces 109 and 603 allow wired communication compliant with a communication standard such as USB, IEEE 1394 (registered trademark), or Ethernet (registered trademark). Like the communication interface 103, the communication interfaces 109 and 603 allow wireless communication compliant with, for example, the Bluetooth (registered trademark) standard and/or the Wi-Fi (registered trademark) standard. The communication interfaces 109 and 603 may be a communication module that allows wireless communication compliant with the Bluetooth Low Energy (BLE) or Low Power Wide Area (LPWA) communication technology.

It should be noted that the communication interfaces 103 and 603 may be used to allow communication between the first user's terminal device 100 and the bicycle trainer 60. In that case, the first user's terminal device 100 may not include the communication interface 109.

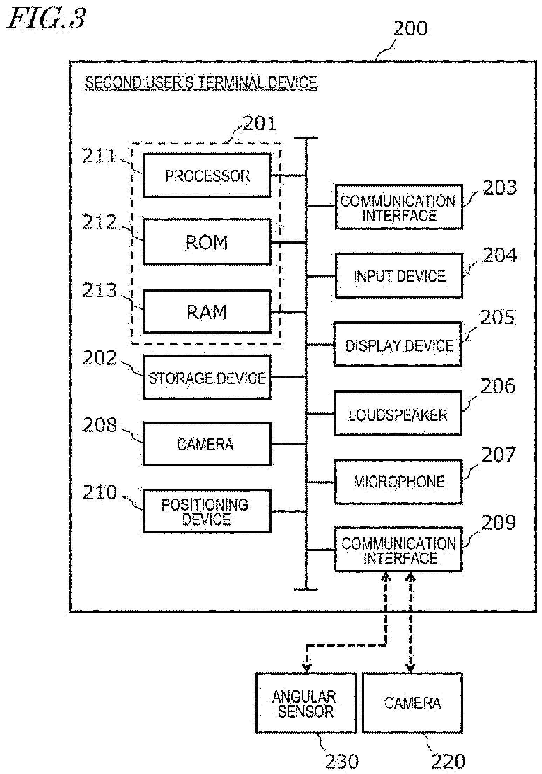

Next, an example of a hardware configuration of the second user's terminal device 200 will be described. FIG. 3 is a block diagram illustrating an example of a hardware configuration of the second user's terminal device 200. The second user's terminal device 200 is, for example, a smartphone, tablet computer, or bicycle computer.

The second user's terminal device 200 includes a processing device 201, a storage device 202, a communication interface (second communication device) 203, an input device 204, a display device 205, a loudspeaker 206, a microphone 207, a camera 208, a communication interface 209, and a positioning device 210. These constituent elements are connected through a bus so as to communicate with each other.

The processing device 201 includes a processor 211, a ROM 212, and a RAM 213. The processor 211, the ROM 212, the RAM 213, and the storage device 202 similar to the processor 111, the ROM 112, the RAM 113, and the storage device 102 of the first user's terminal device 100 and therefore will not herein be described.

The communication interface 203 is a communication module for communication with the first user's terminal device 100 through the network 40. The communication interface 203 may allow wireless communication that uses mobile telephony or satellite communications. In the case in which a mobile router is provided on the bicycle 21 or carried by the second user, the communication interface 203 may be connected to the mobile router by wired or wireless communication. In that case, the communication interface 203 is connected to the communication network 40 through the mobile router.

The input device 204 is for converting instructions from the second user 20 into data, and inputting the data into the computer. The input device 204 is, for example, a touch panel. The display device 205 is, for example, a liquid crystal display or OLED display.

The loudspeaker 206 converts an audio signal into sound, and outputs the sound. The microphone 207 converts sound around the second user's terminal device 200 into an audio signal. The loudspeaker 206 and the microphone 207 can be used as a voice communication device that allows a voice communication with the first user 10. The camera 208 captures an image of a part of surroundings around the second user's terminal device 200 to generate an image signal. The camera 208 is configured such that the lens thereof can be aimed in any direction. For example, the camera 208 can capture an image of the second user 20 by aiming the lens at the second user 20. As described below, the camera 208 can capture an image of a landscape of an area where the second user 20 is riding the bicycle 21 by aiming the lens in the forward direction of the bicycle 21. The camera 208 may be a device that is capable of capturing a 360-degree image of an area around the second user's terminal device 200.

The positioning device 210 can detect a position (geographic coordinates) of the bicycle 21 in a geographic coordinate system. The positioning device 210 receives a GNSS signal transmitted from a GNSS satellite, and performs positioning based on the GNSS signal. GNSS collectively refers to satellite-based positioning systems, such as the global positioning system (GPS), the quasi-zenith satellite system (QZSS, for example, Michibiki), GLONASS, Galileo, and BeiDou. Positioning may be performed by any technique that can obtain positional information having a required precision. The positioning technique may, for example, be interference positioning or relative positioning.

The communication interface 209 is a communication module for communication with the camera 220 and the angular sensor 230. The communication interface 209 communicates with the camera 220 and the angular sensor 230 through wired communication and/or wireless communication. The camera 220 is, for example, attached to the handlebar 24 with the lens thereof aimed in the forward direction of the bicycle 21, and captures an image of a landscape of an area where the second user 20 is riding the bicycle 21. The angular sensor 230 outputs a signal corresponding to an inclination of the bicycle 21. The output signals of the camera 220 and the angular sensor 230 are input to the processor 211 through the communication interface 209.

It should be noted that the second user's terminal device 200 may communicate with the camera 220 through the communication interface 203. Likewise, the second user's terminal device 200 may communicate with the angular sensor 230 through the communication interface 203. In those cases, the second user's terminal device 200 may not include the communication interface 209. In the case in which the camera 208 is used to capture an image of a landscape of an area where the second user 20 is riding the bicycle 21, the camera 220 may be removed. The angular sensor 230 may be built in the second user's terminal device 200.

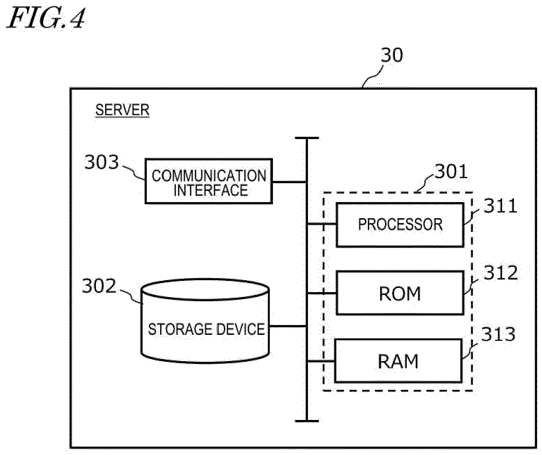

Next, an example of a hardware configuration of the server 30 will be described. FIG. 4 is a block diagram illustrating an example of a hardware configuration of the server 30. The server 30 may be a computer that is located away from the first user 10 and the second user 20, such as a cloud server or edge server. The server 30 includes a processing device 301, a storage device 302, and a communication interface 303. These constituent elements are connected through a bus so as to communicate with each other. The server 30 serves as a cloud server that manages information about the exercise system 1 in a centralized manner, and provides exercise to the first user 10 and the second user 20 by utilizing the managed data.

The processing device 301 includes a processor 311, a ROM 312, and a RAM 313. The processor 311, the ROM 312, the RAM 313, and the storage device 302 are similar to the processor 111, the ROM 112, the RAM 113, and the storage device 102 of the first user's terminal device 100 and therefore will not herein be described.

The communication interface 303 is a communication module for communication with the first user's terminal device 100 and the second user's terminal device 200 through the network 40. The communication interface 303 allows wired communication and/or wireless communication. Like the communication interface 103, the communication interface 303 allows wired communication compliant with a communication standard such as USB, IEEE 1394 (registered trademark), or Ethernet (registered trademark). Like the communication interface 103, the communication interface 303 allows wireless communication compliant with, for example, the Bluetooth (registered trademark) standard and/or the Wi-Fi (registered trademark) standard.

Next, an operation of the exercise system 1 will be described in detail. As described above, the exercise system 1 allows the first user 10 who uses the exercise device 11 in the indoor place 70 and the second user 20 who moves outdoors to do exercise simultaneously. For example, the first user 10 is doing exercise, i.e., rotating the pedals 56 of the bicycle 50, in the indoor place 70, and at the same time, the second user 20 is doing exercise, i.e., riding the bicycle 21 on the road surface 86, rotating the pedals 26, outdoors. The exercise system 1 provides, to the first user 10, a virtual experience that allows the first user 10 to feel as if the first user 10 were moving outdoors together with the second user 20.

In the exercise system 1, an image is displayed which includes a virtual positional relationship between the first user 10 and the second user 20 in an outdoor area where the second user 20 is moving. The first user 10 who is doing exercise using the exercise device 11 in the indoor place 70 can have a virtual experience as if the first user 10 were moving outdoors together with the second user 20, by viewing an image including the virtual positional relationship.

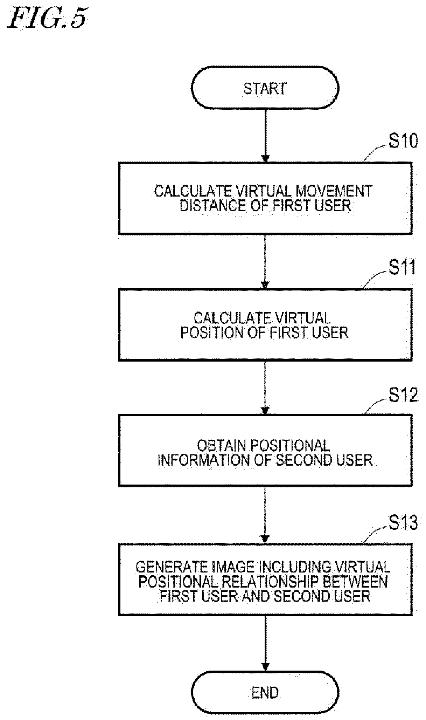

FIG. 5 is a flowchart illustrating an example of a process of generating an image including the virtual positional relationship in an outdoor area between the first user 10 and the second user 20.

The process illustrated in FIG. 5 is mainly executed by the processing device 101 (FIG. 2) of the first user's terminal device 100. At least a portion of the process of FIG. 5 may be executed by the processing device 301 (FIG. 4) of the server 30.

As described above, the rotational sensor 605 (FIG. 2) of the bicycle trainer 60 is provided at any position on the power transmission path between the electric motor 61 and the pedals 56. The rotational sensor 605, which is provided on the power transmission path, detects the angle of rotation of the power transmission path, which is rotated by the first user 10 rotating the pedals 56 of the bicycle 50, and outputs a signal corresponding to that angle of rotation. In other words, the rotational sensor 605 outputs a signal corresponding to a rotation that is produced by the motion of rotating the pedals 56. The processor 611 of the bicycle trainer 60 outputs the output signal of the rotational sensor 605 to the first user's terminal device 100.

The processor 111 of the first user's terminal device 100 calculates the number of revolutions of a rear wheel that is virtually mounted on the bicycle 50, from the output signal of the rotational sensor 605. The ROM 112 or the storage device 102 stores preset information about the size of the rear wheel. For example, the first user 10 can previously input the size of the rear wheel using the input device 104.

The processor 111 calculates a virtual movement distance of the first user 10 using the calculated number of revolutions of the rear wheel and the information about the size of the rear wheel (step S10). The processor 111 can also calculate a virtual travel speed of the first user 10 using the calculated number of revolutions of the rear wheel and the information about the size of the rear wheel.

Next, the processor 111 calculates a virtual position in an outdoor area of the first user 10 from the virtual movement distance of the first user 10 (step S11). For example, the second user 20 takes a preset travel route. The virtual position of the first user can be calculated based on the virtual movement distance of the first user from a predetermined position (e.g., the starting point) on the preset route. Geographic coordinates of the virtual position of the first user 10 can be calculated using information about geographic coordinates of the travel route.

The second user's terminal device 200 which is mounted on the bicycle 21 which moves outdoors includes the positioning device 210 (FIG. 3). The positioning device 210 detects geographic coordinates of the position of the bicycle 21 (i.e., the position of the second user 20). The processor 211 of the second user's terminal device 200 generates positional information indicating the geographic coordinates of the position of the second user 20 from the output signal of the positioning device 210, and transmits the positional information to the first user's terminal device 100 through the network 40.

The first user's terminal device 100 obtains the positional information of the second user 20 from the second user's terminal device 200 through the network 40 (step S12).

The processor 111 generates an image including the virtual positional relationship in an outdoor area between the first user 10 and the second user 20, using the geographic coordinates of the virtual position of the first user and the geographic coordinates of the position of the second user 20, and displays the image on the display device 105 (step S13).

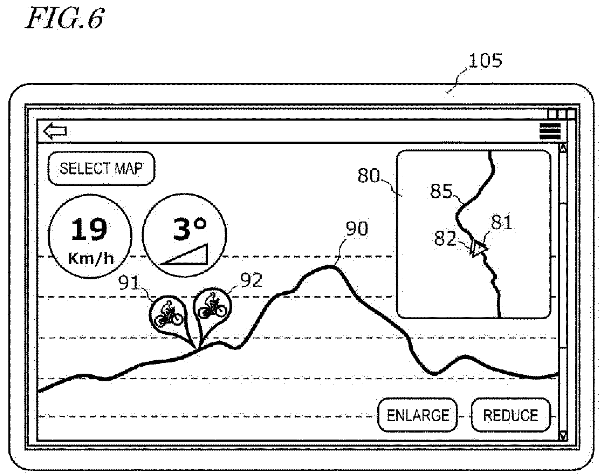

FIG. 6 is a diagram illustrating an example of the image including the virtual positional relationship in an outdoor area between the first user 10 and the second user 20.

The image displayed on the display device 105 of FIG. 6 includes images of a map 80 and an altitude graph 90.

The storage device 302 of the server 30 previously stores map information and altitude graph information of an outdoor area where the second user 20 will move. The map information and the altitude graph information include information about geographic coordinates of an area indicated by a map and an altitude graph. The processor 111 downloads the map information and the altitude graph information from the server 30, and stores these pieces of information in the storage device 102 or the ROM 112.

The processor 111 associates the virtual geographic coordinates of the first user 10 and the geographic coordinates of the second user 20 with the map information and the altitude graph information, to generate images of the map 80 and the altitude graph 90 visually indicating the positions of the first user 10 and the second user 20.

In the example of FIG. 6, the map 80 includes a travel route 85, an icon 81 indicating the position of the first user 10, and an icon 82 indicating the position of the second user 20. The altitude graph 90 includes an icon 91 indicating the position of the first user 10 and an icon 92 indicating the position of the second user 20. A positional relationship between the icon 81 and the icon 82 represents a virtual positional relationship in an outdoor area between the first user 10 and the second user 20. A positional relationship between the icon 91 and the icon 92 represents a virtual positional relationship in an outdoor area between the first user 10 and the second user 20.

The first user 10 who is doing exercise using the exercise device 11 in the indoor place 70 can have a virtual experience as if the first user 10 were moving outdoors together with the second user 20, by viewing an image including a virtual positional relationship in an outdoor area between the first user 10 and the second user 20.

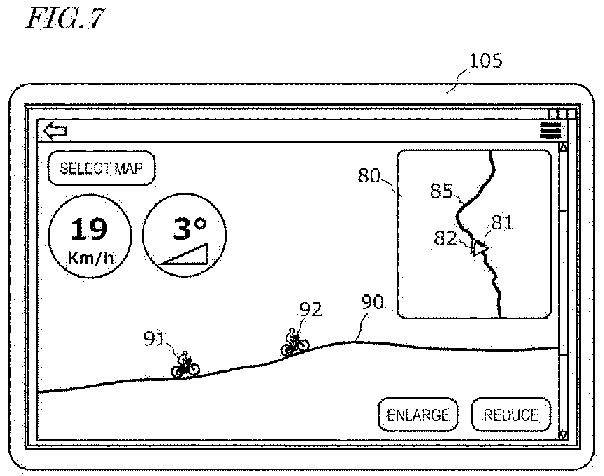

The map 80 and the altitude graph 90 can be enlarged and reduced. FIG. 7 is a diagram illustrating an example of an enlarged image of a portion of the altitude graph 90. The altitude graph 90 can be used as a map obtained when the travel route is viewed horizontally. The enlargement of a portion of the altitude graph 90 can allow more clear presentation of a virtual positional relationship between the first user 10 and the second user 20. If a portion of the map 80 is enlarged, a virtual positional relationship between the first user 10 and the second user 20 can be more clearly shown.

Next, another example of an image including a virtual positional relationship between the first user 10 and the second user 20 will be described.

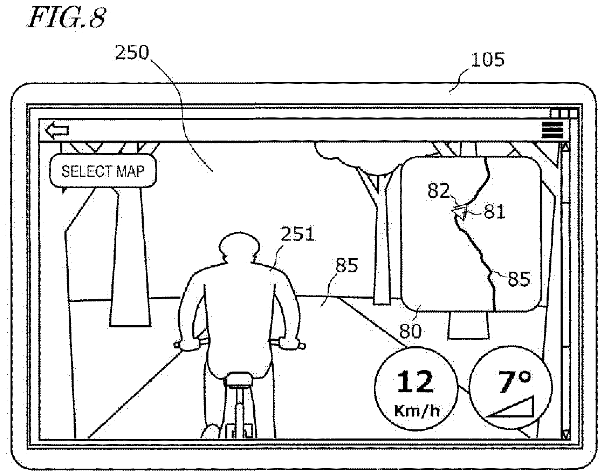

FIG. 8 is a diagram illustrating another example of an image including a virtual positional relationship in an outdoor area between the first user 10 and the second user 20. An image displayed on the display device 105 of FIG. 8 includes a landscape-related image 250 related to a landscape of an outdoor area. Examples of the landscape-related image 250 include a direct image of a landscape of an outdoor area captured by a camera, and an animation image related to a landscape of an outdoor area.

The storage device 302 of the server 30 previously stores image information indicating landscape-related images 250 related to landscapes at a plurality of positions in an outdoor area. The image information includes information about geographic coordinates of positions associated with the landscape-related images 250. The processor 111 downloads the image information from the server 30, and stores the image information in the storage device 102 or the ROM 112.

The processor 111 associates the virtual geographic coordinates of the first user 10 and the geographic coordinates of the second user 20 with the image information. The processor 111 displays the landscape-related image 250 associated with the virtual geographic coordinates of the first user 10 on the display device 105. The processor 111 also displays, on the display device 105, the landscape-related image 250 together with an image of an avatar 251 of the second user 20, based on a virtual positional relationship between the first user 10 and the second user 20.

Thus, an image corresponding to a landscape that could be viewed by the first user 10 if the first user 10 were actually moving outdoors is displayed on the display device 105, whereby a more realistic experience can be provided.

The processor 111 also changes a display position and display size of an image of the avatar 251 of the second user 20, depending on a virtual positional relationship between the first user 10 and the second user 20.

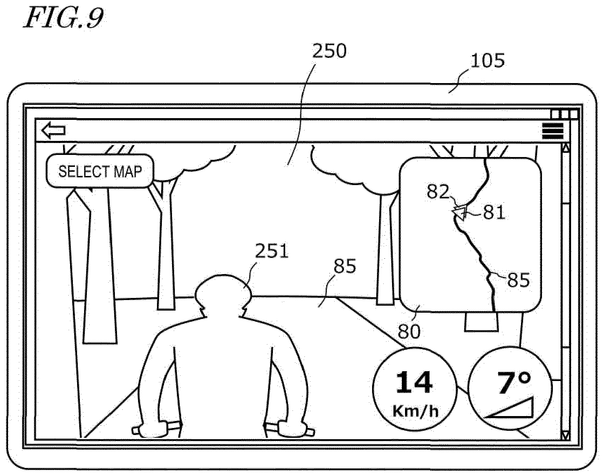

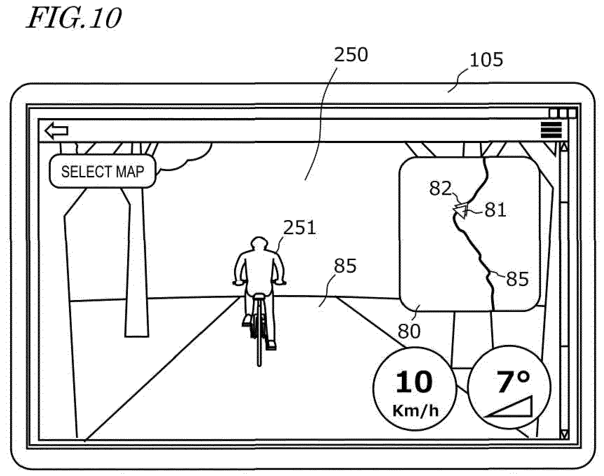

FIG. 9 is a diagram illustrating the avatar 251 when the second user 20 is located at a position that is a short distance in front of the first user 10. FIG. 10 is a diagram illustrating the avatar 251 when the second user 20 is located at a position that is a long distance in front of from the first user 10. When the second user 20 is located at a position that is a short distance in front of the first user 10, the image of the avatar 251 is enlarged, and is displayed at a closer position. When the second user 20 is located at a position that is a long distance in front of the first user 10, the image of the avatar 251 is reduced, and is displayed at a further position.

Such changes in position and size of the displayed avatar 251 of the second user 20 allow the first user 10 to intuitively understand a virtual positional relationship between the first user 10 and the second user 20.

The landscape-related image 250 may be live video. The camera 220 mounted on the bicycle 21 which moves outdoors can capture an image of a landscape of an outdoor area where the second user 20 is riding the bicycle 21. The camera 220 outputs image information of the captured landscape to the second user's terminal device 200. The processor 211 of the second user's terminal device 200 adds, to the image information of the captured landscape, information about geographic coordinates detected by the positioning device 210, and transmits the resultant information to the first user's terminal device 100 through the network 40.

The first user's terminal device 100 receives the image information of the captured landscape from the second user's terminal device 200 through the network 40. The processor 111 associates the virtual geographic coordinates of the first user 10 and the geographic coordinates of the second user 20 with the image information. The processor 111 displays, on the display device 105, the landscape-related image 250 associated with the virtual geographic coordinates of the first user. The processor 111 also displays, on the display device 105, the image of the avatar 251 of the second user 20, whose display position and display size are changed, depending on a virtual positional relationship between the first user 10 and the second user 20, together with the landscape-related image 250.

Thus, an image of the current landscape associated with the virtual position of the first user 10 is displayed on the display device 105, whereby a more realistic experience can be provided.

In addition, when the landscape-related image 250 is displayed, an image of an avatar of the first user 10 may be displayed in addition of the image of the avatar 251 of the second user 20. When both of the avatars of the first user 10 and the second user 20 are displayed, the first user 10 can easily recognize a virtual positional relationship between the first user 10 and the second user 20.

As described above, the loudspeaker 106 and the microphone 107 (FIG. 2) of the first user's terminal device 100 can be used as a voice communication device for a voice communication with the second user 20. The loudspeaker 206 and the microphone 207 (FIG. 3) of the second user's terminal device 200 can be used as a voice communication device for a voice communication with the first user 10.

The first user 10 and the second user 20 can have a voice communication through the first user's terminal device 100 and the second user's terminal device 200. As a result, the first user 10 and the second user 20 can do exercise while having a conversation with each other. The first user 10 can also have a voice communication with the second user 20 while viewing the images illustrated in FIGS. 6 to 10, whereby a more realistic experience can be provided.

Next, an embodiment in which a load applied to the first user 10 is changed, depending on the slope angle of the road surface, will be described.

The electric motor 61 included in the bicycle trainer 60 (FIG. 1) can be used as an adjustment device that adjusts the magnitude of a load applied to the first user 10. The electric motor 61 generates a torque to increase the load applied to the first user 10. The torque generated by the electric motor 61 is transmitted to the sprocket 63 through the power transmission mechanism 62, so that a load can be applied to the first user 10.

The storage device 302 of the server 30 (FIG. 4) previously stores slope angle information indicating the slope angles of road surfaces 86 at a plurality of positions on the travel route 85 on which the second user 20 will move. The slope angle information includes information about the geographic coordinates of the positions of the road surfaces 86 associated with the slope angles. The processor 111 downloads the slope angle information from the server 30, and stores the slope angle information in the storage device 102 or the ROM 112.

The processor 111 associates the virtual geographic coordinates of the first user 10 with the slope angle information. The processor 111 outputs an instruction value for driving the electric motor 61 to the bicycle trainer 60 such that the load applied to the first user 10 is changed, depending on the slope angle of a predetermined position on the travel route 85 associated with the virtual position of the first user 10.

The processor 111 of the bicycle trainer 60 causes the drive device 604 to generate a drive current corresponding to the instruction value. The electric motor 61, when supplied with the drive current, generates a torque. By the torque generated by the electric motor 61, a load can be applied to the first user 10.

For example, the electric motor 61 is controlled such that the load applied to the first user 10 increases with an increase in the slope angle at the virtual position of the first user 10. For example, the electric motor 61 is controlled such that the load applied to the first user 10 increases with an increase in the slope angle at the virtual position of the first user 10 of an ascending slope. Thus, the first user 10 virtually experiences a load that would be applied if the first user 10 were actually traveling outdoors, whereby a more realistic experience can be provided.

The load applied to the first user 10 may also be adjusted using information about a slope angle detected in real time. A method of adjusting the load applied to the first user 10 using information about a slope angle detected in real time will be described below.

The angular sensor 230 (FIG. 3) mounted on the bicycle 21 that moves outdoors outputs a signal corresponding to an inclination of the bicycle 21. The processor 211 of the second user's terminal device 200 calculates the slope angle of the road surface 86 on which the second user 20 is riding the bicycle 21, from the output signal of the angular sensor 230, to generate slope angle information indicating the calculated slope angle. The processor 211 associates the slope angle information with geographic coordinates detected by the positioning device 210, and transmits the resultant information to the first user's terminal device 100 through the network 40.

As partially described above, the first user's terminal device 100 obtains information in which the slope angle information is associated with geographic coordinates, from the second user's terminal device 200, through the network 40. Based on the obtained information, the processor 111 identifies the slope angle at a predetermined position on the travel route 85 associated with the virtual position of the first user 10, and outputs, to the bicycle trainer 60, an instruction value for causing the electric motor 61 to drive according to the slope angle. Specifically, the processor 111 outputs an instruction value to the bicycle trainer 60 such that the magnitude of the load applied to the first user 10 is changed, depending on the identified slope angle.

It should be noted that the processor 211 may transmit, to the first user's terminal device 100, only the slope angle information separately from geographic coordinates detected by the positioning device 210, without associating the slope angle information with the geographic coordinates. In that case, the first user's terminal device 100 may associate the received slope angle information with geographic coordinates, and output, to the bicycle trainer 60, an instruction value corresponding to the position of the first user 10 on the travel route 85.

Such a configuration allows the first user 10 to virtually experience a load that is applied to the second user 20 when the second user 20 is currently riding the bicycle 21 outdoors, whereby a more realistic experience can be provided.

The slope angle of the road surface may be calculated using altitude information included in the positional information of the second user 20. In the case in which the positioning device 210 (FIG. 3) is capable of detecting the altitude of the position of the second user 20, the processor 211 of the second user's terminal device 200 generates positional information indicating the geographic coordinates and altitude of the position of the second user 20 from the output signal of the positioning device 210.

The processor 111 of the first user's terminal device 100 extracts, from the positional information of the second user 20, the geographic coordinates and the altitude information indicating the altitude of the position of the second user 20. The processor 111 calculates a horizontal length between two predetermined positions on the travel route 85 on which the second user 20 moves, by calculating the difference between the geographic coordinates of the two predetermined positions. The processor 111 also calculates an altitude difference of the two predetermined positions by calculating the difference between the altitudes of the two predetermined positions. The processor 111 can calculate the slope angle of the road surface using the calculated length and altitude difference. By sequentially performing such calculation on a plurality of sets of two positions, the slope angles of road surfaces at a plurality of positions on the travel route 85 can be obtained. The value of the slope angle of the road surface can be calculated and obtained using the altitude information without using the angular sensor 230.

In the case of descending slopes, the electric motor 61 may be controlled such that the load applied to the first user 10 decreases with an increase in the slope angle of the virtual position of the first user 10. Also, in the case of descending slopes, the virtual position of the first user 10 may be moved forward even when the first user 10 does not rotate the pedals 56.

The load applied to the first user 10 may be adjusted based on a virtual positional relationship between the first user 10 and the second user 20. For example, the processor 111 controls the electric motor 61 such that in the virtual positional relationship between the first user 10 and the second user 20, the load applied to the first user 10 is smaller when the first user 10 is located behind the second user 20 than when ahead of the second user 20. Thus, when the first user 10 is located behind the second user 20, the load applied to the first user 10 is reduced, and therefore, the first user 10 can virtually experience slipstreaming, whereby a more realistic experience can be provided.

Next, an embodiment in which vibration corresponding to a state of the road surface 86 is provided to the first user 10 will be described.

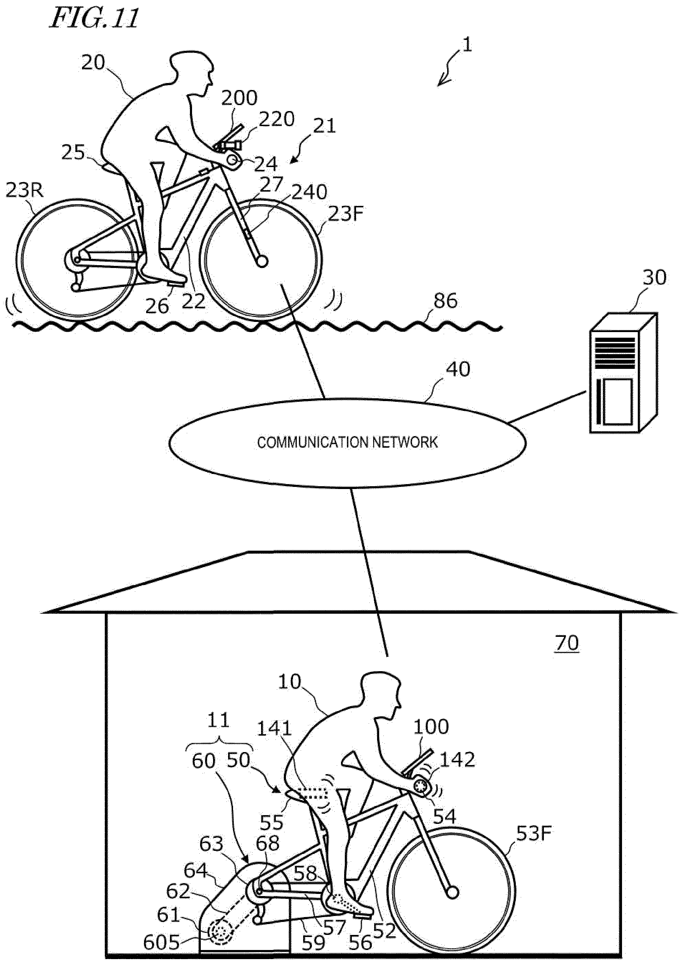

FIG. 11 is a diagram illustrating the exercise system 1 that provides vibration corresponding to a state of the road surface 86 to the first user 10.

The saddle 55 of the bicycle 50 in the indoor place 70, on which the first user 10 sits, is provided with a vibration generation device 141 that generates vibration. The handlebar 54, which the first user 10 holds by their hands, is provided with a vibration generation device 142. The vibration generation devices 141 and 142 are capable of communicating with the communication interface 109 of the first user's terminal device 100 through wired communication and/or wireless communication. The bicycle 50 may be provided with only one of the vibration generation devices 141 and 142.

The storage device 302 of the server 30 previously stores vibration information that is associated with a plurality of positions on the travel route 85 on which the second user 20 will move. The travel route 85 may have road surfaces that cause relatively large vibration of a bicycle, such as roughly paved road surfaces and stone-paved road surfaces. The vibration information includes information about vibration of a bicycle that is generated, depending on the state of the road surface. The processor 111 downloads the vibration information from the server 30, and stores the vibration information in the storage device 102 or the ROM 112.

The processor 111 associates the virtual geographic coordinates of the first user 10 with the vibration information. The processor 111 outputs an instruction value for generating vibration to the vibration generation devices 141 and 142, based on the vibration information of a predetermined position on the travel route 85 associated with the virtual position of the first user 10. The vibration generation devices 141 and 142 generate vibration corresponding to the instruction value.

The first user 10 virtually experiences vibration that would occur if the first user 10 were actually traveling outdoors, whereby a more realistic experience can be provided.

The vibration generation devices 141 and 142 may also be allowed to generate vibration using information about vibration that is detected in real time. The bicycle 21 (FIG. 11) of the second user 20 is provided with a vibration sensor 240. The position where the vibration sensor 240 is provided is not particularly limited. In the example of FIG. 11, the vibration sensor 240 is mounted on a front fork 27.

The vibration sensor 240 mounted on the bicycle 21 that moves outdoors outputs a signal corresponding to vibration of the bicycle 21. The processor 211 of the second user's terminal device 200 generates vibration sensor output information from the output signal of the vibration sensor 240. The processor 211 adds information about geographic coordinates detected by the positioning device 210 to the vibration sensor output information to generate vibration information. The processor 211 transmits the generated vibration information to the first user's terminal device 100 through the network 40.

The first user's terminal device 100 obtains the vibration information from the second user's terminal device 200 through the network 40. The processor 111 associates the virtual geographic coordinates of the first user 10 with the vibration information. The processor 111 outputs, to the vibration generation devices 141 and 142, an instruction value for generating vibration based on the vibration information of a predetermined position on the travel route 85 which is associated with the virtual position of the first user 10. The vibration generation devices 141 and 142 generate vibration corresponding to the instruction value.

The first user 10 can virtually experience vibration that is felt by the second user 20 when the second user 20 is currently riding the bicycle 21 outdoors, whereby a more realistic experience can be provided.

Next, an embodiment in which the first user 10 is caused to virtually experience wind that is felt by the second user 20 when the second user 20 is currently riding the bicycle 21 outdoors will be described.

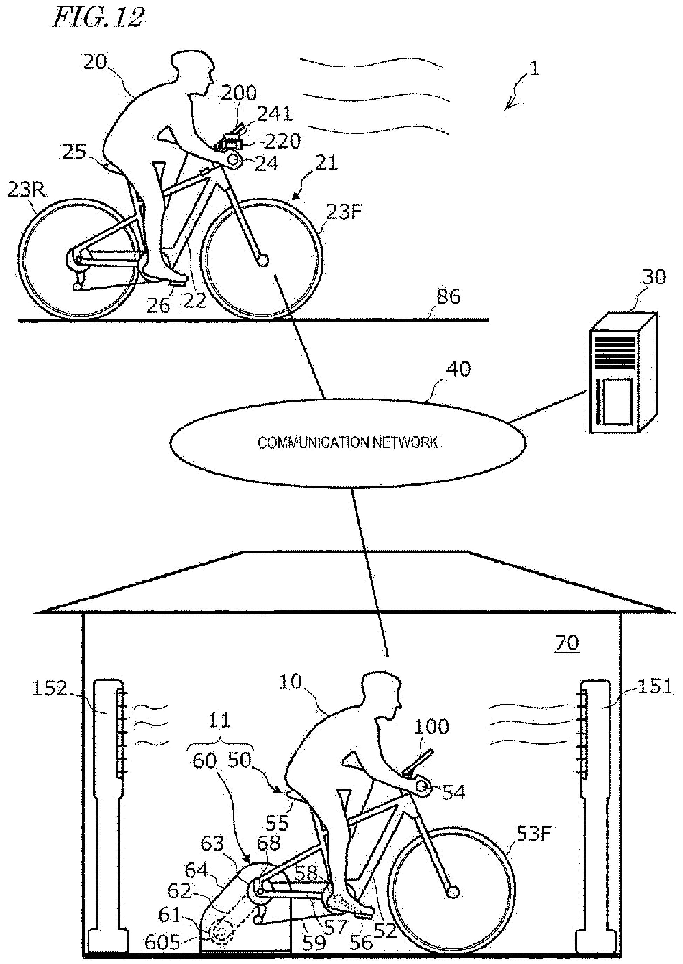

FIG. 12 is a diagram illustrating the exercise system 1 that allows the first user 10 to virtually experience wind that is felt by the second user 20 when the second user 20 is currently riding the bicycle 21 outdoors. The bicycle 21 (FIG. 12) that the second user 20 rides is provided with a wind speed sensor 241. The position where the wind speed sensor 241 is provided is not particularly limited. In the example of FIG. 12, the wind speed sensor 241 is mounted on the handlebar 24. The wind speed sensor 241 outputs a signal corresponding to wind striking the bicycle 21. The wind striking the bicycle 21 corresponds to wind that strikes the body of the second user 20. The wind speed sensor 241 may be mounted on both of a front and a rear portion of the bicycle 21.

Blowers 151 and 152 that create airflow (wind) are provided for the first user 10. In the example of FIG. 12, the blower 151 is disposed in front of the exercise device 11 in the indoor place 70. The blower 152 is disposed at the back of the exercise device 11 in the indoor place 70. The blower 151 is capable of creating airflow blowing from in front of the first user 10 (headwind). The blower 152 is capable of creating airflow from behind the first user 10 (tailwind). The blowers 151 and 152 can communicate with the communication interface 109 of the first user's terminal device 100 through wired communication and/or wireless communication. Only one of the blowers 151 and 152 may be provided in the indoor place 70.

The wind speed sensor 241 mounted on the bicycle 21 which moves outdoors outputs a signal corresponding to wind striking the bicycle 21. The processor 211 of the second user's terminal device 200 generates wind speed information from the output signal of the wind speed sensor 241, and transmits the wind speed information to the first user's terminal device 100 through the network 40.

The first user's terminal device 100 obtains the wind speed information from the second user's terminal device 200 through the network 40. The processor 111 outputs an instruction value for generating wind to the blowers 151 and 152, based on the wind speed information. The blowers 151 and 152 create airflow corresponding to the instruction value.

The first user 10 can virtually experience wind that is felt by the second user 20 when the second user 20 is currently riding the bicycle 21 outdoors, whereby a more realistic experience can be provided.

The load applied to the first user 10 may also be changed, depending on the wind speed information associated with wind striking the second user 20 and the bicycle 21. A load corresponding to wind striking the second user 20 and the bicycle 21 is applied to the second user 20 who is riding the bicycle 21 outdoors. The first user 10 virtually experiences such a load applied to the second user 20, whereby a more realistic experience can be provided.

The processor 111 controls a load (torque) that is generated in the electric motor 61, based on the wind speed information. The load generated by the electric motor 61 is transmitted to the pedals 56 rotated by the first user 10, and therefore, can be felt by the first user 10. For example, if the load generated by the electric motor 61 is increased with an increase in the wind speed of the headwind, a load corresponding to the wind speed of the headwind can be applied to the first user 10. In addition, for example, if the load generated by the electric motor 61 is decreased with an increase in the wind speed of the tailwind, a load corresponding to the wind speed of the tailwind can be applied to the first user 10. The load applied to the first user 10 is thus changed, depending on the wind speed, whereby a more realistic experience can be provided.

Next, information that is presented to the second user 20 will be described.

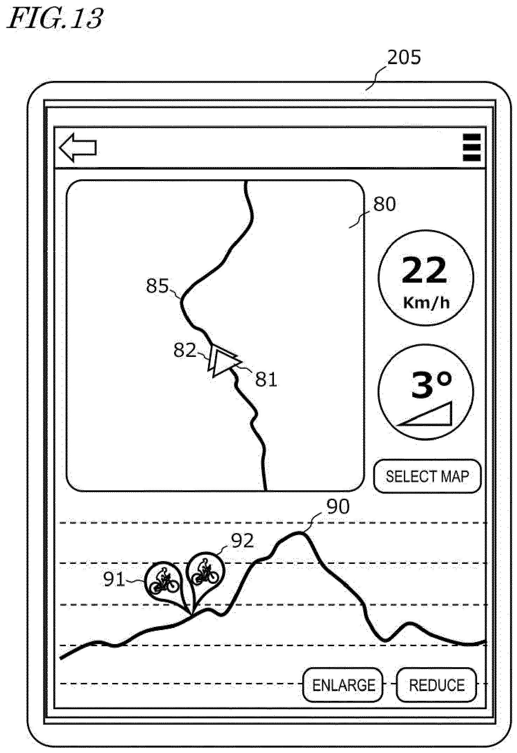

In the embodiment described above, an image including a virtual positional relationship in an outdoor area between the first user 10 and the second user 20 is presented to the first user 10. Such an image may be presented to the second user 20. FIG. 13 is a diagram illustrating an example of an image that is displayed on the display device 205 of the second user's terminal device 200. The image displayed on the display device 205 shows a virtual positional relationship in an outdoor area between the first user 10 and the second user 20. The image displayed on the display device 205 of FIG. 13 includes images of a map 80 and an altitude graph 90. The map 80 displays a travel route 85, an icon 81, and an icon 82. The altitude graph 90 shows an icon 91 and an icon 92. A positional relationship between the icon 81 and the icon 82 represents a virtual positional relationship in an outdoor area between the first user 10 and the second user 20. A positional relationship between the icon 91 and the icon 92 represents a virtual positional relationship in an outdoor area between the first user 10 and the second user 20.

For example, image information of the map 80 and the altitude graph 90 generated by the first user's terminal device 100 may be received by the second user's terminal device 200, and may be displayed on the display device 205.

Alternatively, images of the map 80 and the altitude graph 90 may be generated by the second user's terminal device 200, and may be displayed on the display device 205.

As described above with reference to FIG. 5, the processor 111 of the first user's terminal device 100 calculates the virtual movement distance of the first user 10. The processor 111 transmits information about the virtual movement distance of the first user 10 to the second user's terminal device 200 through the network 40. The processor 211 of the second user's terminal device 200 calculates the geographic coordinates of the virtual position in an outdoor area of the first user 10, based on the information about the virtual movement distance of the first user 10.

Alternatively, the processor 111 of the first user's terminal device 100 may calculate and transmit the geographic coordinates of the virtual position in an outdoor area of the first user 10, to the second user's terminal device 200 through the network 40.

The positioning device 210 of the second user's terminal device 200 detects the geographic coordinates of the position of the second user 20.

The processor 211 generates an image including a virtual positional relationship in an outdoor area between the first user 10 and the second user 20, using the geographic coordinates of the virtual position of the first user 10 and the geographic coordinates of the position of the second user 20, and displays the image on the display device 205.

The storage device 302 of the server 30 previously stores map information and altitude graph information of an outdoor area where the second user 20 will move. The processor 211 downloads the map information and the altitude graph information from the server 30, and stores the map information and the altitude graph information in the storage device 102 or the ROM 112.

The processor 211 associates the virtual geographic coordinates of the first user 10 and the geographic coordinates of the second user 20 with the map information and the altitude graph information, to generate images of the map 80 and the altitude graph 90 which visually indicate the positions of the first user 10 and the second user 20.

The map 80 shows the travel route 85, the icon 81 indicating the position of the first user 10, and the icon 82 indicating the position of the second user 20. The altitude graph 90 shows the icon 91 indicating the position of the first user 10 and the icon 92 indicating the position of the second user 20. A positional relationship between the icon 81 and the icon 82 represents a virtual positional relationship in an outdoor area between the first user 10 and the second user 20. A positional relationship between the icon 91 and the icon 92 represents a virtual positional relationship in an outdoor area between the first user 10 and the second user 20.

The second user 20 can compare the virtual position of the first user 10 with their position in an outdoor area, by viewing the image including a virtual positional relationship between the first user 10 and the second user 20. As a result, the second user 20 can have an experience as if the second user 20 were moving outdoors together with the first user 10.

Avatars as described with reference to FIGS. 8 to 10 may be displayed on the display device 205 of the second user's terminal device 200. In that case, only the avatar of the first user 10 may be displayed on the display device 205, or both of the avatars of the first user 10 and the second user 20 may be displayed. By viewing the images of the avatars, the second user 20 can easily recognize a virtual positional relationship between the first user 10 and the second user 20, and can have an experience as if the second user 20 were moving outdoors together with the first user 10.

Next, an embodiment in which a load applied to the second user 20 is changed will be described.

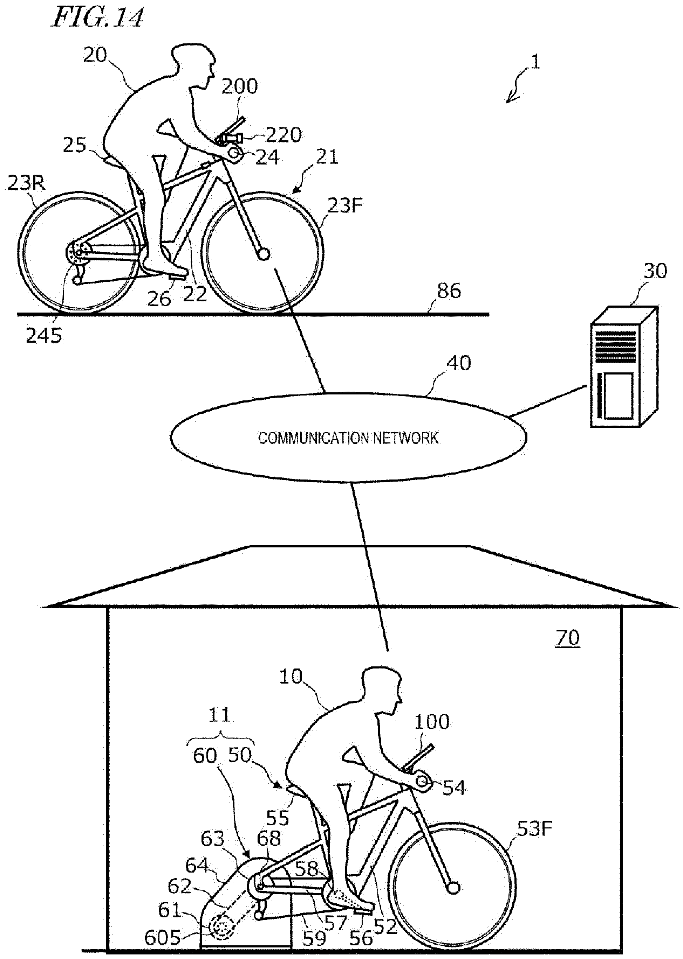

FIG. 14 is a diagram illustrating the exercise system 1 in which a load applied to the second user 20 is changed. The bicycle 21 of the second user 20 of FIG. 14 is provided with an adjustment device 245 that adjusts a load applied to the second user 20. The adjustment device 245 is capable of communicating with the communication interface 209 of the second user's terminal device 200 through wired communication and/or wireless communication. In the example of FIG. 14, the adjustment device 245 is a hub motor that is coaxial with the rear wheel 23R. The hub motor 245 generates a torque that is used as a load applied to the second user 20.

As described above, the electric motor 61 of the first user 10 generates a torque that is used as a load applied to the first user 10.

For example, loads applied to the first user 10 and the second user 20 may be changed, depending on a state in a game that is played by the first user 10 and the second user 20. The processor 111 controls the electric motor 61 such that the magnitude of the load applied to the first user 10 is changed, depending on a state in a game that is played by the first user 10 and the second user 20. The processor 211 controls the hub motor 245 such that the load applied to the second user 20 is changed, depending on a state in the game that is played by the first user 10 and the second user 20. For example, the electric motor 61 and the hub motor 245 may be controlled such that the load applied to the winner is decreased while the load applied to the loser is increased.

If the loads applied to the first user 10 and the second user 20 are changed, depending on a state in a game, the first user 10 and the second user 20 can do exercise while enjoying playing the game.

Next, an embodiment will be described in which when the first user 10 is virtually traveling on a curve, the virtual travel speed of the first user 10 is limited to a preset upper limit value or less.

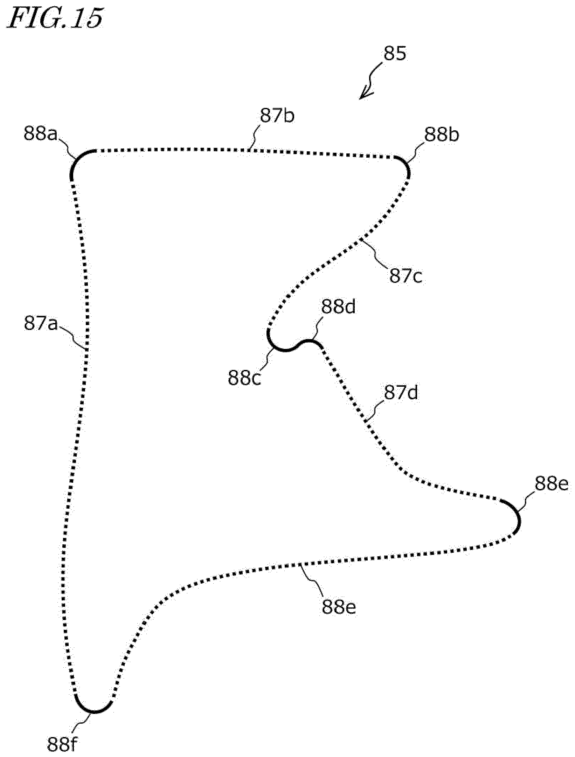

FIG. 15 is a diagram illustrating an example of a preset travel route 85 on which the first user 10 and the second user 20 do exercise simultaneously. The travel route 85 includes straight line portions 87 and curved portions 88.

In this embodiment, the curved portion 88 is defined as a portion of the travel route 85 whose radius of curvature is smaller than or equal to a predetermined radius of curvature R0. The straight line portion 87 is defined as a portion of the travel route 85 whose radius of curvature is greater than the predetermined radius of curvature R0. The straight line portion 87 is not limited to an exact straight line route, and includes a mildly curved portion on which a bicycle can travel without deceleration. The curved portion 88 is not limited to an arc that extends along a circumstantial portion of a perfect circle, and may, for example, be a portion whose average radius of curvature is smaller than or equal to the predetermined radius of curvature R0. The predetermined radius of curvature R0 is, for example, but not limited to, 20 to 30 m.

The radius of curvature of each portion of the travel route 85 is, for example, obtained as follows. A third party carrying a positioning device previously obtains geographic coordinates of road surfaces at a plurality of positions on the travel route 85 while moving on the travel route 85. Thereafter, from the plurality of geographic coordinates thus obtained, the radius of curvature of each portion of the travel route 85 is calculated, whereby information about the radii of curvature can be obtained. The radius of curvature may, for example, be one that is calculated at a point at or near the center in the transverse direction of a road surface. A road surface of the travel route 85 can be classified as a straight line portion 87 or a curved portion 88, based on information about the radius of curvature of that portion of the travel route 85.

In FIG. 15, the straight line portions 87 are indicated by a dotted line, and the curved portions 88 are indicated by a solid line. The travel route 85 of FIG. 15 includes straight line portions 87a, 87b, 87c, 87d, and 87e as the straight line portions 87, and curved portions 88a, 88b, 88c, 88d, 88e, and 88f as the curved portions 88. For each curved portion 88, the upper limit value of travel speed is previously set. The upper limit value of travel speed for each curved portion 88 is, for example, the maximum speed at which a bicycle can travel on that curved portion, and which is set, depending on the radius of curvature of that curved portion.

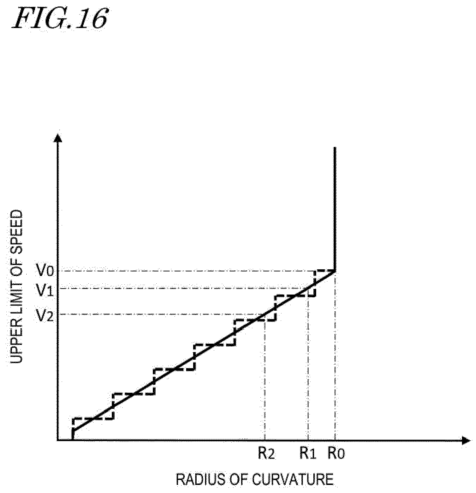

FIG. 16 is a diagram illustrating an example of a relationship between the radius of curvature and the upper limit value of travel speed. In FIG. 16, the vertical axis represents the upper limit value of travel speed, and the horizontal axis represents the radius of curvature.

For the straight line portions 87, i.e., portions whose radii of curvature are greater than the predetermined radius of curvature Ro, the upper limit value of travel speed is not set. For the curved portions 88, i.e., portions whose radii of curvature are smaller than or equal to the predetermined radius of curvature Ro, the upper limit value of travel speed is set. For example, as indicated by a solid line in FIG. 16, the upper limit value of travel speed is decreased with a decrease in the radius of curvature. For each curved portion 88, the upper limit value of travel speed is set, corresponding to the radius of curvature of that portion. In the case in which the radius of curvature is equal to the predetermined radius of curvature Ro, the upper limit value V₀ of travel speed is, for example, but not limited to, 35 to 45 km/h.

Map information of the travel route 85 that includes the straight line portions 87 and the curved portions 88 is previously stored in the storage device 302 of the server 30 (FIG. 4). The map information of the travel route 85 includes information about a preset upper limit value of travel speed for each curved portion 88. The processor 111 downloads the map information of the travel route 85 from the server 30, and stores the map information of the travel route 85 in the storage device 102 or the ROM 112.

If the first user 10 is doing exercise and is virtually positioned on a curved portion 88, the processor 111 performs control such that the virtual travel speed of the first user 10 is lower than or equal to the upper limit value of travel speed set for that curved portion 88. For example, the processor 111 performs control such that the virtual movement distance per unit time of the first user 10 is lower than or equal to the movement distance per unit time taken when the travel speed is equal to the upper limit value.

Curved portions of actual roads have an upper limit value at which a bicycle can travel. When a bicycle travels on a curved portion, the travel speed of the bicycle is decelerated to the upper limit value or less. In this embodiment, when the first user 10 is virtually positioned on a curved portion 88, the virtual travel speed of the first user 10 is set to be lower than or equal to the preset upper limit value of that curved portion 88. For example, when the virtual position of the first user 10 is moved from a straight line portion 87 to a curved portion 88, then if the virtual travel speed is greater than the upper limit value, the virtual travel speed is controlled to be lower than or equal to the upper limit value. As a result, the first user 10 can have an experience similar to traveling on an actual curve, whereby a more realistic experience can be provided.

When the virtual position of the first user 10 is moved from a straight line portion 87 to a curved portion 88, then if the virtual travel speed at that time is lower than or equal to the upper limit value, it is determined that the first user 10 can travel on that curved portion 88, keeping that travel speed, and therefore, the control of reducing the virtual travel speed may not be performed.

When the first user 10 is virtually positioned on a curved portion 88, then even if the first user 10 rotates the pedals 56, the virtual travel speed is limited to the preset upper limit value or less. The virtual position of the first user 10 can be inhibited from being moved on a curved portion at a travel speed that would cause the first user 10 to go off that curved portion if the first user 10 were traveling on an actual road. Thus, the first user 10 can have an experience similar to traveling on an actual curve, whereby a more realistic experience can be provided.

When the first user 10 who is present in the indoor place 70 and the second user 20 who is present outdoors compete against each other in a race, then if the virtual travel speed of the first user 10 is reduced on curved portions where deceleration is required so that the second user 20 does not suffer from a disadvantage when the second user 20 decelerates on actual curved portions, the fairness of the race can be increased.

As described above, the upper limit value of travel speed is decreased with a decrease in the radius of curvature of a curved portion 88. For example, the radius of curvature R2 of the curved portion 88b is smaller than the radius of curvature R1 of the curved portion 88a. In this case, the upper limit value V₂ of travel speed of the curved portion 88b is set to be smaller than the upper limit value V₁ of travel speed of the curved portion 88a. Thus, the upper limit value of travel speed is reduced to a greater extent for a curved portion 88 having a smaller radius of curvature, and therefore, the first user 10 can have an experience similar to traveling on actual curves, whereby a more realistic experience can be provided.

The relationship between the radius of curvature of a curved portion 88 and the upper limit value of travel speed set therefor may be changed continuously as indicated by the solid line in FIG. 16 or discretely as indicated by the dashed line in FIG. 16.

A curved portion 88 may include a portion that is closer to the first user 10 in the virtual travel direction than is a portion whose radius of curvature is smaller than or equal to the predetermined radius of curvature R₀. For example, a curved portion 88 may include a portion that is closer to the first user 10 by 5 to 10 m than is a portion whose radius of curvature is smaller than or equal to the predetermined radius of curvature R₀. When the virtual position of the first user 10 is approaching a portion having a small radius of curvature, deceleration can be started, and therefore, the first user 10 can have an experience similar to traveling on an actual curve, whereby a more realistic experience can be provided.

The virtual travel speed of the first user 10 with respect to the operation amount of the exercise device 11 used by the first user 10 may be lower when the first user 10 is virtually positioned on the curved portions 88 than when on the straight line portions 87. In the case of traveling on actual roads, the travel speed is typically lower on curves than on straight lines. If the virtual travel speed with respect to the operation amount of the exercise device 11 is lower when the first user 10 is virtually positioned on the curved portions 88 than when on the straight line portions 87, the first user 10 can have an experience similar to a change in speed during traveling on an actual road.

As described above, the exercise device 11 is provided with the adjustment device (electric motor) 61 that adjusts the magnitude of the load applied to the first user 10. The processor 111 may control the electric motor 61 such that the load applied to the first user 10 is greater when the second user 20 who is preset outdoors is positioned on the curved portions 88 than when on the straight line portions 87. The second user 20 who is traveling on the actual outdoor route 85 may take a lower travel speed on curves than on straight lines. When the first user 10 who is present in the indoor place 70 and the second user 20 who is present outdoors compete against each other in a race, then if the load applied to the first user 10 is increased so that the second user 20 does not suffer from a disadvantage when the second user 20 decelerates on the curved portions 88, the fairness of the race can be increased.

The timing at which a load is applied to the first user 10 may not be the same as the timing at which the second user 20 is positioned on a curved portion 88. The electric motor 61 may be controlled such that the total amount of loads that are applied to the first user 10 when the first user 10 virtually moves from the start to finish of the travel route 85 is equal to a predetermined total load amount. In that case, the map information of the travel route 85 may include information about the preset total load amount.

The timing at which a load is applied to the first user 10 for increase of fairness is not particularly limited. The application of a load to the first user 10 can be inhibited from being performed at a timing unnatural to the first user 10. For example, a great load can be inhibited from being applied to the first user 10 when the first user 10 is virtually traveling on the straight line portions 87, and therefore, the first user 10 can enjoy themselves in a race with natural feeling.

In the case in which the magnitude of the load applied to the first user 10 is adjusted so as to increase fairness as described above, the upper limit value of travel speed may not be set for the curved portions 88.

Although, in the embodiment described above, the exercise device 11 is an indoor cycling machine, the exercise device 11 may be a treadmill. Treadmills are an exercise device for running and/or walking indoors. In that case, the second user 20 who is present outdoors may run or walk, carrying the second user's terminal device 200. In the case of running or walking, images as described above may be displayed on the first user's terminal device 100, so that the first user 10 can have a virtual experience as if the first user 10 were moving outdoors together with the second user 20. In addition, images as described above may be displayed on the second user's terminal device 200, so that the second user 20 can have a virtual experience as if the second user 20 were moving outdoors together with the first user 10.

In the embodiment described above, the first user 10 and the second user 20 do exercise. According to the present teaching, three or more users can do exercise simultaneously, and share the information described above. As an example, a third user who moves outdoors may do exercise simultaneously in addition to the first user 10 and the second user 20, and the first to third users may do exercise while sharing information. The third user may do exercise indoors.

The third user may not do exercise. For example, the third user may be a team manager or spectator, and the first user 10 and the second user 20 may do exercise while communicating with the third user. When the third user is present near the first user 10, the third user may communicate with the second user 20 through the first user's terminal device 100. When the third user is present near the second user 20, the third user may communicate with the first user 10 through the second user's terminal device 200.

An icon and/or avatar of the third user may be included in an image including a virtual positional relationship between the users described with reference to FIGS. 6 to 10 and 13. Likewise, in the case in which there are four or more participants, an icon and/or avatar of each user may be included in an image including a virtual positional relationship between the users. As a result, a virtual positional relationship between all of the participants can be easily recognized, and the participants can have an experience as if all of them moved together outdoors.

In the foregoing, some illustrative embodiments of the present teaching have been described.

An exercise system 1 according to an embodiment of the present teaching in which a first user 10 who uses an exercise device 11 in an indoor place 70 and a second user 20 who moves outdoors do exercise simultaneously, includes: for the first user 10, an exercise device 11 that is used by the first user 10 in the indoor place 70; a first sensor 605 that outputs a signal corresponding to an operation of the exercise device 11 produced by the first user 10 using the exercise device 11; a first processing device 101 that calculates a virtual movement distance of the first user 10 based on the output signal of the first sensor 605; and a display device 105 that presents information to the first user 10; and for the second user 20, a second processing device 201 that obtains and outputs positional information indicating geographic coordinates of a position of the second user 20 who moves outdoors, wherein the first processing device 101 displays, on the display device 105, an image 80, 90, 250 including a virtual positional relationship between the first user 10 and the second user 20 in an outdoor area where the second user 20 is moving, based on the positional information of the second user 20 and the virtual movement distance of the first user 10.

The first user 10 who does exercise using the exercise device 11 in the indoor place 70 can view the image including the virtual positional relationship between the first user 10 and the second user 20 in an outdoor area where the second user 20 is moving, and therefore, can have a virtual experience as if the first user 10 were moving outdoors together with the second user 20.

In an embodiment, the first processing device 101 may display, on the display device 105, a map 80, 90 showing the position of the second user 20 and a virtual position of the first user 10, based on map information of the outdoor area where the second user 20 is moving, the positional information of the second user 20, and the virtual movement distance of the first user 10.

The first user 10 can recognize the virtual positional relationship between the first user 10 and the second user 20 in the outdoor area by viewing the map indicating the position of the second user 20 and the virtual position of the first user 10.

In an embodiment, the first processing device 101 may obtain image information indicating landscape-related images 250 related to landscapes at a plurality of positions in the outdoor area, calculate a virtual position of the first user 10 in the outdoor area from the virtual movement distance of the first user 10, and display, on the display device 105, a landscape-related image 250 in association with the virtual position of the first user 10, together with an image of an avatar 251 of the second user 20 whose display position and display size are changed, depending on the virtual positional relationship between the first user 10 and the second user 20.

Thus, images corresponding to landscapes that could be viewed by the first user 10 if the first user 10 were actually moving outdoors are displayed on the display device 105, whereby a more realistic experience can be provided.

Thus, the position and size of the displayed avatar 251 of the second user 20 are changed, whereby the first user 10 can intuitively recognize the virtual positional relationship between the first user 10 and the second user 20.

In an embodiment, the second processing device 201 may output image information of landscapes of the outdoor area captured during movement of the second user 20, and the first processing device 101 may display an image of a landscape associated with the virtual position of the first user 10 on the display device 105.

Thus, an image of a current landscape associated with the virtual position of the first user 10 is displayed on the display device 105, whereby a more realistic experience can be provided.

In an embodiment, the first processing device 101 may display, on the display device 105, an image of an avatar 251 of the second user 20 whose display position and display size are changed, depending on the virtual positional relationship between the first user 10 and the second user 20.

Thus, the position and size of the displayed avatar 251 of the second user 20 are changed, whereby the first user 10 can intuitively recognize the virtual positional relationship between the first user 10 and the second user 20.

In an embodiment, the second user 20 may move on a preset travel route 85, and the first processing device 101 may calculate a virtual position of the first user 10 on the preset travel route 85 from the virtual movement distance of the first user 10.

As a result, the virtual position of the first user 10 can be easily calculated.

In an embodiment, the exercise device 11 used by the first user 10 may be for indoor cycling.

Thus, the first user 10 who is present in the indoor place 70 can have a virtual experience as if the first user 10 were moving outdoors together with the second user 20 while riding a bicycle.

In an embodiment, the second user 20 may move outdoors while riding a bicycle 21.

Thus, the first user 10 who is present in the indoor place 70 can have a virtual experience as if the first user 10 were cycling outdoors together with the second user 20.

In an embodiment, the exercise device 11 may be provided with an electric motor (adjustment device) 61 that adjusts the magnitude of a load applied to the first user 10. The first processing device 101 may obtain slope angles of road surfaces at a plurality of positions on a travel route 85 on which the second user 20 moves, calculate a virtual position of the first user 10 on the travel route 85 from the virtual movement distance of the first user 10, and control the adjustment device 61 such that the load applied to the first user 10 is changed, depending on the slope angle at a predetermined position on the travel route 85 associated with the virtual position of the first user 10.

Thus, the first user 10 virtually experiences a load that would be applied if the first user 10 were actually traveling outdoors, whereby a more realistic experience can be provided.

In an embodiment, the bicycle 21 that the second user 20 rides may be provided with an angular sensor 230 that outputs a signal corresponding to an inclination of the bicycle 21. The second processing device 201 may calculate a slope angle of the travel route 85 on which the second user 20 is moving, from the output signal of the angular sensor 230, and output slope angle information indicating the calculated slope angle. The first processing device 101 may obtain a slope angle at the predetermined position from the slope angle information.

Thus, the first user 10 can virtually experience a load that is applied to the second user 20 when the second user 20 is currently traveling outdoors, whereby a more realistic experience can be provided.

In an embodiment, the positional information of the second user 20 may include altitude information indicating an altitude of the position of the second user 20. The first processing device 101 may calculate a length between two predetermined positions on the travel route 85 on which the second user 20 moves, based on geographic coordinates of the two predetermined positions, calculate an altitude difference between the two predetermined positions based on the altitude information of the two predetermined positions, and calculate a slope angle of the road surface based on the calculated length and altitude difference.

Thus, the slope angle of a road surface is calculated using the altitude information, whereby the value of the slope angle can be obtained without using the angular sensor 230.

In an embodiment, the exercise device 11 may be provided with an electric motor (adjustment device) 61 that adjusts the magnitude of a load applied to the first user 10. The first processing device 101 may control the adjustment device 61 such that the load applied to the first user 10 is smaller when the first user 10 is positioned behind the second user 20 than when ahead of the second user 20 in the virtual positional relationship between the first user 10 and the second user 20.

Thus, the load applied to the first user 10 is reduced to a greater extent when the first user 10 is positioned behind the second user 20, and therefore, the first user 10 can virtually experience slipstreaming, whereby a more realistic experience can be provided.

In an embodiment, the exercise device 11 may be provided with a saddle 55 on which the first user 10 sits, a handlebar 54 that is held by the hands of the first user 10, and a vibration generation device 141, 142 that vibrates at least one of the saddle 55 and the handlebar 54. The first processing device 101 may obtain vibration information associated with a plurality of positions on a travel route 85 on which the second user 20 moves, calculate a virtual position of the first user 10 on the travel route 85 from the virtual movement distance of the first user 10, and control the vibration generated by the vibration generation device 141, 142, based on the vibration information of a predetermined position on the travel route 85 associated with the virtual position of the first user 10.

Thus, the first user 10 virtually experiences vibration that would occur if the first user 10 were actually traveling outdoors, whereby a more realistic experience can be provided.

In an embodiment, the bicycle 21 that the second user 20 rides may be provided with a vibration sensor 240 that outputs a signal corresponding to vibration of the bicycle 21. The second processing device 201 may generate vibration sensor output information from the output signal of the vibration sensor 240, and output the vibration sensor output information. The first processing device 101 may associate the vibration sensor output information with the positional information of the second user 20 to generate the vibration information.

Thus, the first user 10 can virtually experience vibration that is felt by the second user 20 when the second user 20 is currently traveling outdoors, whereby a more realistic experience can be provided.

In an embodiment, a blower 151, 152 that creates airflow may be provided for the first user 10, the bicycle 21 of the second user 20 may be provided with a wind speed sensor 241 that outputs a signal corresponding to wind striking the bicycle 21 of the second user 20 or the second user 20. The second processing device 201 may generate wind speed information from the output signal of the wind speed sensor 241, and output the wind speed information. The first processing device 101 may control the airflow created by the blower 151, 152, based on the wind speed information.

Thus, the first user 10 can virtually experience wind that is felt by the second user 20 when the second user 20 is currently traveling outdoors, whereby a more realistic experience can be provided.

In an embodiment, the exercise device 11 may be provided with an electric motor (adjustment device) 61 that adjusts the magnitude of a load applied to the first user 10, and the bicycle 21 of the second user 20 may be provided with a wind speed sensor 241 that outputs a signal corresponding to wind striking the bicycle 21 of the second user 20 or the second user 20. The second processing device 201 may generate wind speed information from the output signal of the wind speed sensor 241, and output the wind speed information. The first processing device 101 may control the adjustment device 61 such that the magnitude of the load applied to the first user 10 is changed, depending on the wind speed information.

Thus, the first user 10 can virtually experience a load corresponding to wind that is felt by the second user 20 when the second user 20 is currently traveling outdoors, whereby a more realistic experience can be provided.

In an embodiment, the second processing device 201 may display information that is presented to the second user 20 on the display device 205. The first processing device 101 may output information about the virtual movement distance of the first user 10. The second processing device 201 may calculate a virtual position of the first user 10 in the outdoor area based on the information about the virtual movement distance of the first user 10, and display an image showing the virtual position of the first user 10 on the display device 205.

Thus, the second user 20 can compare their own position with the virtual position of the first user 10 in the outdoor area by viewing the image including the virtual position of the first user 10. As a result, the second user 20 can experience as if the second user 20 moved outdoors together with the first user 10.

In an embodiment, the second processing device 201 may display information that is presented to the second user 20 on the display device 205. The first processing device 101 may calculate a virtual position of the first user 10 in the outdoor area based on the virtual movement distance of the first user 10, and output information about the virtual position of the first user 10. The second processing device 201 may display an image showing the virtual position of the first user 10 on the display device 205 based on the information about the virtual position of the first user 10.

Thus, the second user 20 can compare their own position with the virtual position of the first user 10 in the outdoor area by viewing the image including the virtual position of the first user 10. As a result, the second user 20 can experience as if the second user 20 moved outdoors together with the first user 10.

In an embodiment, the first processing device 101 and the second processing device 201 may each include a voice communication device 106, 107, 206, 207. The first user 10 and the second user 20 may be allowed to have a voice communication through the first processing device 101 and the second processing device 201.

Thus, the first user 10 and the second user 20 can do exercise while having a conversation with each other.

In an embodiment, the exercise device 11 used by the first user 10 may be provided with an electric motor (adjustment device) 61 that adjusts the magnitude of a load applied to the first user 10. The first processing device 101 may control the adjustment device 61 such that the magnitude of the load applied to the first user 10 is changed, depending on a state in a game that is played by the first user 10 and the second user 20.

Thus, the load applied to the first user 10 is changed, depending on a state in a game, whereby the first user 10 can do exercise while enjoying playing the game.

In an embodiment, the bicycle 21 that the second user 20 rides may be provided with an adjustment device 245 that adjusts the magnitude of a load applied to the second user 20. The second processing device 201 may control the adjustment device 245 provided on the bicycle 21 such that the magnitude of the load applied to the second user 20 is changed, depending on a state in a game that is played by the first user 10 and the second user 20.

Thus, the load applied to the second user 20 is changed, depending on a state in a game, whereby the second user 20 can do exercise while enjoying playing the game.

In an embodiment, the preset travel route 85 may include a curved portion 88. For the curved portion 88, an upper limit value of travel speed may be previously set. The first processing device 101 may perform control such that a virtual travel speed of the first user 10 is lower than or equal to the upper limit value when a virtual position of the first user 10 is on the curved portion 88.

The speed at which traveling is possible on a curved portion 88 of a road has an upper limit. The speed at which traveling is possible on a curved portion 88 is, for example, previously set based on the radius of curvature R of the curved portion 88.

The virtual travel speed of the first user 10 on a curved portion 88 is set to be lower than or equal to a preset upper limit value. For example, when the virtual position of the first user 10 is moved from a straight line portion 87 to a curved portion 88, then if the virtual travel speed is greater than the upper limit value, the virtual travel speed is controlled to be lower than or equal to the upper limit value. As a result, the first user 10 can have an experience similar to traveling on an actual curve, whereby a more realistic experience can be provided.

The virtual position of the first user 10 can be inhibited from being moved on a curved portion at a travel speed that would cause the first user 10 to go off that curved portion if the first user 10 were traveling on an actual road. Thus, the first user 10 can have an experience similar to traveling on an actual curve, whereby a more realistic experience can be provided.

When the first user 10 who is present in an indoor place 70 and the second user 20 who is present outdoors compete against each other in a race, then if the virtual travel speed of the first user 10 is reduced on curved portions where deceleration is required, the fairness of the race can be increased.

In an embodiment, the preset travel route 85 may include a first curved portion 88a and a second curved portion 88b. A radius of curvature R₂ of the second curved portion 88b may be smaller than a radius of curvature R₁ of the first curved portion 88a, and a preset upper limit value V₂ of travel speed of the second curved portion 88b may be smaller than a preset upper limit value of travel speed V₁ of the first curved portion 88a.

Thus, the upper limit value of travel speed is reduced to a greater extent for a curved portion 88 having a smaller radius of curvature R, and therefore, the first user 10 can have an experience similar to traveling on actual curves, whereby a more realistic experience can be provided.

In an embodiment, the preset travel route 85 may include a straight line portion 87 and a curved portion 88. The first processing device 101 may set a virtual travel speed of the first user 10 with respect to an operation amount of the exercise device 11 to be lower when a virtual position of the first user 10 is on the curved portion 88 than when on the straight line portion 87.

In the case of traveling on actual roads, the travel speed is typically lower on curves than on straight lines. If the virtual travel speed with respect to the operation amount of the exercise device 11 is smaller when the first user 10 is virtually positioned on the curved portion 88 than on the straight line portion 87, the first user 10 can have an experience similar to a change in speed during traveling on an actual road.

In an embodiment, the exercise device 11 may be provided with an adjustment device 61 that adjusts the magnitude of a load applied to the first user 10. The preset travel route 85 may include a straight line portion 87 and a curved portion 88. The first processing device 101 may control the adjustment device 61 such that the load applied to the first user 10 is greater when the position of the second user 20 is on the curved portion 88 than when on the straight line portion 87.

The second user 20 who travels on an actual outdoor route may take a lower travel speed on curves than on straight lines. When the first user 10 who is present in an indoor place 70 and the second user 20 who is present outdoors compete against each other in a race, then if the load applied to the first user 10 is increased so that the second user 20 does not suffer from a disadvantage when the second user 20 decelerates on the curved portion 88, the fairness of the race can be increased.

In an embodiment, the exercise device 11 may be provided with an adjustment device 61 that adjusts the magnitude of a load applied to the first user 10. For the preset travel route 85, the total amount of loads applied to the first user 10 may be previously set. The first processing device 101 may control the adjustment device 61 such that the total amount of loads that are applied to the first user 10 when a virtual position of the first user 10 moves from the start to finish of the preset travel route 85 is equal to a predetermined total load amount.

The timing at which a load is applied to the first user 10 for increase of fairness is not particularly limited. The application of a load to the first user 10 can be inhibited from being performed at a timing unnatural to the first user 10.

A method for providing exercise according to an embodiment of the present teaching provides exercise that a first user 10 who uses an exercise device 11 in an indoor place 70 and a second user 20 who moves outdoors do simultaneously. The method includes: calculating a virtual movement distance of the first user 10 based on an operation of the exercise device 11 produced by the first user 10 using the exercise device 11 in the indoor place 70; obtaining positional information indicating geographic coordinates of a position of the second user 20 who moves outdoors; and displaying an image including a virtual positional relationship between the first user 10 and the second user 20 in an outdoor area where the second user 20 is moving, based on the positional information of the second user 20 and the virtual movement distance of the first user 10.

The first user 10 who does exercise using the exercise device 11 in the indoor place 70 can view the image including the virtual positional relationship between the first user 10 and the second user 20 in the outdoor area where the second user 20 is moving, and therefore, can have a virtual experience as if the first user 10 were moving outdoors together with the second user 20.

A computer program according to an embodiment of the present teaching causes a computer to provide exercise that a first user 10 who uses an exercise device 11 in an indoor place 70 and a second user 20 who moves outdoors do simultaneously. The computer program causes the computer to execute: calculating a virtual movement distance of the first user 10 based on an operation of the exercise device 11 produced by the first user 10 using the exercise device 11 in the indoor place 70; obtaining positional information indicating geographic coordinates of a position of the second user 20 who moves outdoors; and displaying an image including a virtual positional relationship between the first user 10 and the second user 20 in an outdoor area where the second user 20 is moving, based on the positional information of the second user 20 and the virtual movement distance of the first user 10.

The first user 10 who does exercise using the exercise device 11 in the indoor place 70 can view the image including the virtual positional relationship between the first user 10 and the second user 20 in the outdoor area where the second user 20 is moving, and therefore, can have a virtual experience as if the first user 10 were moving outdoors together with the second user 20.

The present teaching is particularly useful in the field of systems for doing exercise.

## Claims

1. An exercise system (1) configured for a first user (10) who uses an exercise device (11) indoors and a second user (20) who moves outdoors do exercise simultaneously, comprising:
for the first user (10),
an exercise device (11) that is configured to be used by the first user (10) indoors;
a first sensor (605) that is configured to output a signal corresponding to an operation of the exercise device (11) produced by the first user (10) using the exercise device (11);
a first processing device (101) configured to calculate a virtual movement distance of the first user (10) based on the output signal of the first sensor (605); and
a first display device (105) configured to present information to the first user (10);
a first communication device (103) configured for communication between the first user (10) and the second user (20); and
for the second user (20),
a second processing device (201) configured to obtain and output positional information indicating geographic coordinates of a position of the second user (20) who moves outdoors,
a second communication device (203) configured for communication between the first user (10) and the second user (20), wherein
the first processing device (101) is configured to display, on the first display device (105), an image including a virtual positional relationship between the first user (10) and the second user (20) in an outdoor area where the second user (20) is moving,
based on the positional information of the second user (20) and the virtual movement distance of the first user (10).

2. The exercise system (1) according to claim 1, wherein the first processing device (101) is configured to display, on the first display device (105), a map showing the position of the second user (20) and a virtual position of the first user (10), based on map information of the outdoor area where the second user (20) is moving, the positional information of the second user (20), and the virtual movement distance of the first user (10).

3. The exercise system (1) according to claim 1 or 2, wherein the first processing device (101) is configured to:
obtain image information indicating landscape-related images related to landscapes at a plurality of positions in the outdoor area;
calculate a virtual position of the first user (10) in the outdoor area from the virtual movement distance of the first user (10); and
display, on the first display device (105), a landscape-related image in association with the virtual position of the first user (10), together with an image of an avatar (251) of the second user (20) whose display position and display size are changed, depending on the virtual positional relationship between the first user (10) and the second user (20).

4. The exercise system (1) according to claim 3, wherein the second processing device (201) is configured to output image information of landscapes of the outdoor area captured during movement of the second user (20), and
the first processing device (101) is configured to display an image of a landscape associated with the virtual position of the first user (10) on the first display device (105).

5. The exercise system (1) according to claim 1 or 2, wherein the first processing device (101) is configured to display, on the first display device (105), an image of an avatar (251) of the second user (20) whose display position and display size are changed, depending on the virtual positional relationship between the first user (10) and the second user (20).

6. The exercise system (1) according to any one of claims 1 to 5, wherein when the second user (20) moves on a preset travel route (85), and
the first processing device (101) is configured to calculate a virtual position of the first user (10) on the preset travel route (85) from the virtual movement distance of the first user (10).

7. The exercise system (1) according to any one of claims 1 to 6, wherein the exercise device (11) used by the first user (10) is for indoor cycling.

8. The exercise system (1) according to claim 7, wherein the second user (20) moves outdoors while riding a bicycle (21).

9. The exercise system (1) according to claim 8, wherein the exercise device (11) is provided with an adjustment device (61) configured to adjust the magnitude of a load applied to the first user (10), and
the first processing device (101) is configured to:
obtain slope angles of road surfaces at a plurality of positions on a travel route on which the second user (20) moves;
calculate a virtual position of the first user (10) on the travel route from the virtual movement distance of the first user (10); and
control the adjustment device (61) such that the load applied to the first user (10) is changed, depending on the slope angle at a predetermined position on the travel route associated with the virtual position of the first user (10).

10. The exercise system (1) according to any one of claims 8 to 9, wherein the exercise device (11) is provided with an adjustment device (61) configured to adjust the magnitude of a load applied to the first user (10), and
the first processing device (101) is configured to control the adjustment device (61) such that the load applied to the first user (10) is smaller when the first user (10) is positioned behind the second user (20) than when ahead of the second user (20) in the virtual positional relationship between the first user (10) and the second user (20).

11. The exercise system (1) according to any one of claims 8 to 10, wherein the exercise device (11) is provided with a saddle (55) configured for the first user (10) to sit, a handlebar (54) that is configured to be held by hands of the first user (10), and a vibration generation device (141, 142) that is configured to vibrate at least one of the saddle (55) and the handlebar (54), and
the first processing device (101) is configured to:
obtain vibration information associated with a plurality of positions on a travel route on which the second user (20) moves;
calculate a virtual position of the first user (10) on the travel route from the virtual movement distance of the first user (10); and
control the vibration generated by the vibration generation device (141, 142), based on the vibration information of a predetermined position on the travel route associated with the virtual position of the first user (10).

12. The exercise system (1) according to any one of claims 1 to 11, wherein the second processing device (201) is configured to display information that is presented to the second user (20) on a second display device (205),
the first processing device (101) is configured to output information about the virtual movement distance of the first user (10), and
the second processing device (201) is configured to:
calculate a virtual position of the first user (10) in the outdoor area based on the information about the virtual movement distance of the first user (10); and
display an image showing the virtual position of the first user (10) on the second display device (205).

13. The exercise system (1) according to claim 6, wherein when the preset travel route (85) includes a curved portion (88),
for the curved portion (88), an upper limit value of travel speed is previously set, and the first processing device (101) is configured to perform control such that a virtual travel speed of the first user (10) is lower than or equal to the upper limit value when a virtual position of the first user (10) is on the curved portion (88).

14. The exercise system (1) according to claim 13, wherein when the preset travel route (85) includes a first curved portion (88a) and a second curved portion (88b),
a radius of curvature of the second curved portion (88) is smaller than a radius of curvature of the first curved portion (88), and
a preset upper limit value (V2) of travel speed of the second curved portion (88b) is smaller than a preset upper limit value (V1) of travel speed of the first curved portion (88a).

15. The exercise system (1) according to claim 6 or 13, wherein when the preset travel route (85) includes a straight line portion (87) and a curved portion (88), and
the first processing device (101) is configured to set a virtual travel speed of the first user (10) with respect to an operation amount of the exercise device (11) to be lower when a virtual position of the first user (10) is on the curved portion (88) than when on the straight line portion (87).
